# EUROPEAN PATENT APPLICATION

(11) **EP 3 896 447 A1**
(43) Date of publication of application: **20.10.2021**
(21) Application number: 21168254.7
(22) Date of filing: 14.04.2021
(51) Int. Cl.: G01N 33/543

(54) **A LATERAL FLOW DETECTION DEVICE FOR DETECTING A CORONAVIRUS BY IMMUNOASSAY**

(30) Priority: 16.04.2020 CN 202010300484; 30.04.2020 US 202063018024 P
(71) Applicant: Hangzhou Biotest Biotech Co., Ltd., Hangzhou, Zhejiang 311100 (CN)
(72) Inventor: Ye, Chunsheng, Hangzhou, 311100 (CN); Zhang, Zhenxing, Hangzhou, 311100 (CN); Wang, Zhenyu, Hangzhou, 311100 (CN); Hou, Luna, Hangzhou, 311100 (CN)
(74) Representative: Handley, Matthew Edward

(57) **Abstract**

The invention provides a lateral flow detection device for detecting a coronavirus by immunoassay, wherein the detection device comprises two lateral flow test strips, a test strip 1 is used to test an antibody to a N full-length protein and/or a S full-length protein, while a test strip 2 is used to test an antibody to a S-RBD site protein, and a combination of the two test strips is used to test IgG and IgM antibodies to novel coronavirus, which further improves a detection rate of serological antibodies and effectively reduces a possibility of missing detection and wrong detection, thereby avoiding any missing detection. Simultaneously, the device could be further used to identify an effective antibody produced by an innate immunity and/or after an patient's recovery from disease, so the two test strips are significantly complementary to each other. The detection device of the present invention has the advantages that it detects reliably and efficiently in a simple manner; it can prevent missing detection as possible, it operates conveniently without need of professional instruments and personnel, and easily popularized for clinical promotion and application. Further, the present invention provided two antigen combinations for preparing detection devices for rapid detection of novel coronavirus and an application thereof.

## Description

### Cross -reference

This patent application claims the priority based upon the prior applications of China, with the application serial no: 2020103004847 filed on April 16, 2020; claims the priority based upon a provisional application of US with the application serial no: 63/018,024 filed on April 30, 2020, all the content constitutes a part of the present invention.

### Technical Field

The present invention belongs to the technical field of biological detection, relating to lateral flow detection device for detecting a coronavirus by immunoassay.

### Background Art

A severe cold caused by coronavirus (COVID-19) broke out in Wuhan in 2019. The National Health Commission named the pneumonia caused by the virus infection as novel coronavirus pneumonia (coronavirus disease 2019, COVID-19). COVID-19 epidemic spreads rapidly, quickly spreads across the country and swept the world in just three months. According to the latest reports, more than 2 million cases have been confirmed in more than 200 countries. The high infectivity, high pathogenicity, high severity rate and high fatality rate of such a coronavirus has caused great harm to the global economy, society and health.

As people's knowledge about biological characteristics of novel coronavirus grows, we witnessed certain results have been made in the treatment of COVID-19, and the novel coronary pneumonia epidemic in China is now largely contained. Over 90% of patients in China have been discharged from hospital, but some patients in Guangdong, Sichuan, Hubei, Hunan and other places suffered recurred fever and tested as positive by nucleic acid testing after discharge. There is no definite information to show it attributes to easy discharge standards or recurrence of such virus. The condition of some severe and critical COVID-19 patients are improved after treatment, but they often suffer pulmonary structural changes due to old age, low immune function, structural lung disease, or fibrotic changes of in lung caused by severe condition, and this may result in incomplete blood circulation perfusion, incomplete elimination of some hidden viruses or the cells remaining in a virulent state, but the body's detoxification has not reached the amount of virus required for a positive result in nucleic acid test. Hence, if the body's immunity is low at this time, the virus that has not been completely killed may relapse and reignite, thereby leading to recurrence of a disease. Currently, the asymptomatic carriers pose an enormous challenge to the prevention and control of the epidemic. It becomes a hot issue arousing widely concern in society that whether those COVID-19 asymptomatic carriers will lead to recurrence of novel pneumonia in China.

Currently there are two methods for COVID-19 detection coronary pneumonia, namely nucleic acid testing and immunological detection, wherein the nucleic acid testing has the characteristics of early diagnosis, high sensitivity and specificity, which is called the "gold standard" for diagnosing COVID-19. However, as being affected by high requirements for sample collection, RNA extraction and testing equipment, it may easily cause a false negative result. Therefore, it is of top urgent to develop an immunological detection method to make up for the false negative caused by nucleic acid testing.

2019-nCoV includes a coronavirus spike protein (Spike, S), an envelope protein (Envelope, E), a membrane protein (Membrane, M) and a nucleocapsid protein (Nucleoprotein, N), as well as other structures. S protein can bind to a ACE2 receptor on the surface of a host cell, so it is an important structural protein that mediates a virus to enter into the cell, and also the main antigen that induces neutralizing antibodies.

Usually the S protein can be cleaved into two parts of S1 and S2, wherein S1 mediates virus attachment, S2 mediates membrane fusion, and RBD is the structure of the receptor binding domain bound to the S1 structure.

CN111505277A discloses a detection kit for a novel coronavirus, which is coated with a combination of RBD and N antigens and used for detection of an IgG antibody. RBD as a precise site protein in the kit is combined with a nucleocapsid N protein, which may be prone to cause cross-influence; and it only detects a IgG antibody, thereby further expanding the possibility of missing detection and false detection.

Hence, it is urgent to determine a more suitable antigen combination for preparation of a detection device for rapid detection of a novel coronavirus, in order to further reduce the possibility of missing detection and false detection, and improve detection sensitivity, thereby providing a more reliable site detection means for screening suspected patients and asymptomatic carriers and help preventing the spread of the epidemic.

### Detailed descriptions of the invention

In order to solve the disadvantages of the prior art, the present invention provides a lateral flow detection device for detecting a novel coronavirus by immunoassay which detects reliably and efficient in a simple manner and prevents missing detection as possible; the detection device comprises two lateral flow test strips; wherein a test strip 1 is used to test an antibody to a N full-length protein and/or a S full-length protein, while a test strip 2 is used to test an antibody to a S-RBD site protein, and a combination of the two test strips is used to test IgG and IgM antibodies to novel coronavirus, so that it covers all sites of novel coronavirus that may make the human body produce antibodies and further improves the detection rate of serological antibody detection, thereby avoiding missing detection. Besides, the device detects rapidly and operates conveniently without need of professional instruments and personnel, and easily popularized for clinical promotion and application.

The novel coronavirus first invades a human body by binding of a spike protein S protein on the virus to a ACE2 receptor of human cells; in actual binding, a S protein spiked is not simply inserted into ACE2, while the S protein is cleaved to a S1 subunit and a S2 subunit by a host protease. (Cysteine protease, trypsin, etc.), S1 and S2 fuse with a receptor binding membrane; S1 contains a receptor binding domain RBD, and RBD is the key core for binding to ACE2. Therefore, it can be seen that the antibody to the S-RBD site protein and the antibody to the S full-length protein do not necessarily exist at the same time. Simply detecting the antibody produced by the S-RBD site protein or the antibody produced by the S full-length protein may result in missing detection, so it is very necessary to detect antibodies produced to the S full-length protein and antibodies produced to the S-RBD site protein simultaneously. The S1 subunit can be further divided into two relatively independent domains, namely N-terminal domain (NTD) and C-terminal domain (CTD). S1 contains a receptor binding domain (RBD), and most RBDs of coronavirus S proteins are located in CTD, such as SARS-CoV and MERS-CoV. Only a small part of RBDs of a beta coronavirus are located in NTD (generally NTD binds to carbohydrate receptors and CTD binds to protein receptors). It may be to be concluded from above, if only the antibody to the RBD antigen is detected, it results in missing detection; If the antibody to the full-length S protein is monitored, all the antibodies produced by the virus could be detected more comprehensively.

A N protein is a most abundant protein in the novel coronavirus, which is highly conservative and relates to replication of a viral genome and the regulation of a cell signaling pathway, so the N protein is used as a diagnostic testing tool for novel coronavirus, being core raw material of a rapid immunological diagnostic reagent. However, because the N protein only functions after the novel coronavirus invades a human body, there is still a risk of missing detection if only the antibody to the N full-length protein is detected, it is very necessary to conduct a joint detection of the full-length S protein that functions at the beginning of the invasion, thereby reducing the possibility of missing detection.

A S-RBD site is a receptor binding domain of the novel coronavirus, it is the key core for binding to a ACE2 receptor of human cells, so it is an important fragment to determine an infection of a novel coronavirus. An antibody to the S-RBD site protein is the most efficient among many effective antibodies, simply detecting the antibody to the S-RBD site protein could improve the detection accuracy and efficiency, and this could also be used to identify an effective antibody produced by an innate immunity and/or an immunity after recovery, an enhancing antibody produced may directly act on an RBD fragment in a spike protein, thereby directly preventing a virus from infecting cells. Neither a S full length protein nor N protein has such a function). However, as a precise site protein, if the S-RBD site protein is tested on a test strip with a full-length antigen (such as S or other N antigen), when they are both treated in a detection reaction area, a cross-reaction may be caused and there is no complementary effect to each other. This not only reduces the effect of detecting effective antibodies, but easily leads to missing detection, and moreover, it is impossible to identify an effective antibody produced by an innate immunity and/or an immunity after recovery. In case of the innate immunity, the human body produces an antibody after being infected by a virus, but it may be asymptomatic, the virus remains in the body and may be contagious; the antibody after recovering from such disease indicate that it has been infected by the virus, but thanks to the existing of an antibody, the human body is in a state of recovering.

Unexpectedly, the present invention identifies that, on the condition that the first test strip is used to detect an antibody or a full-length antigen to a N protein (full-length) and/or a S protein (full-length), the second test strip 2 is combined to detect the most effective antibody or RBD antigen produced against the S-RBD site, which produces complementary effects, thereby avoiding any missing detection, and allowing to significantly identify an effective antibody produced by an innate immunity and/or an immunity after recovery.

The said antibody may include IgM or/and IgG antibodies corresponding to the antigens. These antibodies may be present in secretions such as blood, saliva, lungs, and throat, and the said blood includes serum, plasma or whole blood samples. According to relevant studies, after an novel coronavirus invades a human body, an IgM antibody appears in 5-7 days first, and then an IgG antibody appears in 10-15 days. Therefore, an increase in IgM antibody indicates a recent acute infection, and an increase in IgG antibody indicates a previous infection. It can effectively reduce the possibility of missing detection and wrong detection and avoid all missing detections to combine the two antigen proteins in the two test strips provided by the present invention and further jointly detect the IgG and IgM antibodies to novel coronavirus.

Of course, by adjusting an species of antibodies or antigens in the marker area and the detection area, the detection device provided by the present invention could also be used for detection of a novel coronavirus antigen. For example, the RBD antigen is detected on the first test strip, and the N full-length antigen or/and the S full-length antigen is detected on the second test strip. Antibodies for detecting an antigen or other receptors that can bind to the antigen could be used to detect the antigen.

The two antigen combinations described in the present invention may be either natural antigens or recombinant antigens obtained after be expressed by an conventional genetic engineering technology. However, they all need to be detected separately other than being detected together, and the specific meaning of the above separate detection will be explained in details below in the application.

Therefore, the technical scheme provided by the present invention is as follows:
On one hand, the invention provides a lateral flow detection device for detecting a novel coronavirus, wherein the device comprises a first and a second lateral flow test strips, the first lateral flow test strip 1 contains an antibody to a S full-length protein and/or a N full-length protein for detecting a novel coronavirus, while the second lateral flow test strip contains an antibody to a S-RBD site protein for detecting a novel coronavirus. In some embodiments, the said antibody comprises an IgM or/ and IgG antibody.

In some embodiments, the second lateral flow test strip comprises an IgM or/and IgG antibody to a S-RBD site protein for detecting a novel coronavirus.

In some embodiments, the first lateral flow test strip comprises an IgM or/ and IgG antibody to a S full-length protein and/or N full-length protein for detecting a novel coronavirus. In some embodiments, the antibody is an IgM or/ and IgG antibody to a S protein or N protein. In some embodiments, a N protein and a S protein form a detection area. In some embodiments, the IgG to N protein and S protein is detected in the detection area; the IgM to N protein and S protein is detected in another detection area, two detection areas or one of two are located on the same test strip. A reagent for detecting an antibody to an RBD antigen is located on a different test strip, which comprises an IgG or IgM antibody.

An amino acid sequence of the said S full-length protein is indicated by SEQ ID NO.3, and an amino acid sequence of the said N full-length protein is indicated by SEQ ID NO.2, and an amino acid sequence of the said S-RBD site protein is indicated by SEQ ID NO.1.

On the other hand, the invention provides a lateral flow detection device for detecting a novel coronavirus by immunoassay, which comprises a first test strip and a second test strip, wherein the first test strip includes an antigen to a S full-length protein and/or a N full-length protein; and the second test strip includes an antigen to a S-RBD site protein.
1. QHR63250-nCov-S RBD-263aa: 
MPNITNLCPFGEVFNATRFASVYAWNRKRISNCVADYSVLYNSASFSTFKCYGVSPTKLNDLCFTNV
YADSFVIRGDEVRQIAPGQTGKIADYNYKLPDDFTGCVIAWNSNNLDSKVGGNYNYLYRLFRKSNL
KPFERDISTEIYQAGSTPCNGVEGFNCYFPLQSYGFQPTNGVGYQPYRVVVLSFELLHAPATVCGPK
KSTNLVKNKCVNFNFNGLTGTGVLTESNKKFLPFQQFGRDIADTTDAVRDPQTLEILDITPCSFHHHH
HHHH(SEQ ID NO:1).
2. QHN73817-nCov N-419aa: (N full-length protein) 
MHHHHHHSDNGPQNQRNAPRITFGGPSDSTGSNQNGERSGARSKQRRPQGLPNNTASWFTALTQH
GKEDLKFPRGQGVPINTNSSPDDQIGYYRRATRRIRGGDGKMKDLSPRWYFYYLGTGPEAGLPYGA
NKDGIIWVATEGALNTPKDHIGTRNPANNAAIVLQLPQGTTLPKGFYAEGSRGGSQASSRSSSRSRNS
SRNSTPGSSRGTSPARMAGNGGDAALALLLLDRLNQLESKMSGKGQQQQGQTVTKKSAAEASKKP
RQKRTATKAYNVTQAFGRRGPEQTQGNFGDQELIRQGTDYKHWPQIAQFAPSASAFFGMSRIGMEV
TPSGTWLTYTGAIKLDDKDPNFKDQVILLNKHIDAYKTFPPTEPKKDKKKKADETQALPQRQKKQQ
TVTLLPAADLDDFSKQLQQSMSSADSTQA(SEQ ID NO:2).
3. Amino acid sequence of a S full-length protein

1 mfllttkrtmfvflvllplvssqcvnlttrtqlppaytnsftrgvyypdkvfrssvlhst

61 qdlflpffsnvtwfhaihvsgtngtkrfdnpvlpfndgvyfasteksniirgwifgttld
121 sktqsllivnnatnvvikvcefqfcndpflgvyyhknnkswmesefrvyssannctfeyv
181 sqpflmdlegkqgnfknlrefvfknidgyfkiyskhtpinlvrdlpqgfsaleplvdlpi
241 ginitrfqtllalhrsyltpgdsssgwtagaaayyvgylqprtfllkynengtitdavdc
301 aldplsetkctlksftvekgiyqtsnfrvqptesivrfpnitnlcpfgevfnatrfasvy
361 awnrkrisncvadysvlynsasfstfkcygvsptklndlcftnvyadsfvirgdevrqia
421 pgqtgkiadynyklpddftgcviawnsnnldskvggnynylyrlfrksnlkpferdiste
481 iyqagstpcngvegfncyfplqsygfqptngvgyqpyrvvvlsfellhapatvcgpkkst
541 nlvknkcvnfnfngltgtgvltesnkkflpfqqfgrdiadttdavrdpqtleilditpcs
601 fggvsvitpgtntsnqvavlyqdvnctevpvaihadqltptwrvystgsnvfqtragcli
661 gaehvnnsyecdipigagicasyqtqtnsprrarsvasqsiiaytmslgaensvaysnns
721 iaiptnftisvttteilpvsmtktsvdctmyicgdstecsnlllqygsfctqlnraltgia
781 veqdkntqevfaqvkqiyktppikdfggfnfsqilpdpskpskrsfiedllfnkvtlada
841 gfikqygdclgdiaardlicaqkfngltvlppllltdemiaqytsallagtitsgwtfgag
901 aalqipfamqmayrfngigvtqnvlyenqklianqfnsaigkiqdslsstasalgklqdv
961 vnqnaqalntlvkqlssnfgaissvlndilsrldkveaevqidrlitgrlqslqtyvtqq
1021 liraaeirasanlaatkmsecvlgqskrvdfcgkgyhlmsfpqsaphgvvflhvtyvpaq
1081 eknfttapaichdgkahfpregvfvsngthwfvtqrnfyepqiittdntfvsgncdvvig
1141 ivnntvydplqpeldsfkeeldkyfknhtspdvdlgdisginasvvniqkeidrlnevak
1201 nlneslidlqelgkyeqyikwpwyiwlgfiagliaivmvtimlccmtsccsclkgccscg
1261 scckfdeddsepvlkgvklhyt (SEQ ID NO:3)

Further, the said first test strip or the second test strip includes a sample area, a marker area, and a detection area respectively, which are arranged in an order according to a liquid flow direction; wherein a substance coated in the marker area flows with the liquid, and the substance needs couple to a marker substance; the detection area has a test line, and the substance coated in the detection area is fixed on the test line; the said antigen may be coated in the marker area or the detection area.

The substance that flows with the liquid in the marker area is coupled with the marker substance and flows on the test line, and captured by the antibody or antigen on the test line, thus to form a colored line. The substance coated by the test line is fixed. The said marker substance may be fluorescent or colored particles, such as latex, gold particles, or colored water-soluble substances.

Further, the first test strip or the second test strip may also comprise anti-human IgG antibodies and/or anti-human IgM antibodies; and when the antigens are coated in the marker area, the anti-human IgG antibody and/or anti-human IgM antibody are coated in the detection area. Alternatively, when the said antigen is coated in the detection area, the anti-human IgG antibody and/or anti-human IgM antibody are/is coated in the marker area and flow/flows with the sample.

The anti-human IgG antibody and/or anti-human IgM antibody, i.e. the antibody IgM or antibody IgG to be detected, may be a mouse anti-human IgM antibody, a mouse anti-human IgG antibody, a rabbit anti-human IgM antibody or a rabbit anti-human IgG antibody as long as it can specifically capture the antibody IgM or IgG to be detected.

The antigen to S full-length protein and/or N full-length protein on the first test strip may be coated in the marker area and coupled with a marker substance, and after binding to the IgM or IgG in the sample, it flows to the detection area with the liquid, and is then captured by the anti-human IgG antibody and/or anti-human IgM antibody coated on the test line and developed to obtain a test result; on the contrary, when the anti-human IgG antibody and/or anti-human IgM antibody is coated in the marker area and coupled with the marker substance, after binding to the IgM or IgG in the sample, it flows with the liquid to the detection area, and is captured by the S full-length protein antigen and/or N full-length protein antigen coated on the test line and developed to obtain the test result.

Similarly, the antigen to S-RBD site protein on the second test strip could be coated in the marker area and coupled with a marker substance, after binding to the IgM or IgG in the sample, it flows to the detection area with the liquid, and is then captured by the anti-human IgG antibody and/or anti-human IgM antibody coated on the test line and developed to obtain a test result; on the contrary, when the anti-human IgG antibody and/or anti-human IgM antibody is coated in the marker area and coupled with the marker substance, after binding to the IgM or IgG in the sample, it flows with the liquid to the detection area, and is captured by the S-RBD site protein antigen coated on the test line and developed to obtain the test result.

Further, the detection area may have one or more test lines, and the test lines are respectively used for detecting any one or both of IgG and IgM

When a test line is set to test the sum of IgG and IgM, as long as one of IgG or IgM is positive, the result is deemed as positive; when two test lines are set to test IgG and IgM respectively, and one of the two is positive, then the result is deemed as positive; an increase in IgM antibody indicates a recent acute infection, and an increase in IgG antibody indicates a previous infection; when two test lines are set, there is another case in the same test strip that one test line is used to detect antibodies to N full-length protein (including the sum of IgG and IgM), and the other test line is used to detect antibodies to S full-length protein (including the sum of IgG and IgM).

Of course, by adjusting an species of antibodies in the marker area and the detection area, the detection device provided by the present invention could also be used for detection of a novel coronavirus antigen. At this time, one or more test lines can be respectively used to detect different antigens.

Further, the device is characterized in that a S-RBD site protein antigen on the second test strip is coated in the marker area, and the said anti-human IgG antibody and the anti-human IgM antibody are respectively coated on different test lines of the detection area.

The S-RBD site protein antigen in the test strip 2 is coated in the marker area, and the anti-human IgG antibody and anti-human IgM antibody are respectively coated on different test lines in the detection area and used to detect IgG and IgM respectively, which not only prevents missing detection, but also effectively identifies an effective antibody produced by an innate immunity and/or an immunity after recovery, thereby further exerting the function of the test strip 2.

Further, on the said first test strip, when the first antibody of the S full-length protein and/or the N full-length protein antibody are/is coated in the marker area, the second antibody of the antibody to S full-length protein and/N full-length protein can be immobilized in the detection area, or the second antibody of the first antibody to S full-length protein and/N full-length protein can be immobilized in the detection area.

The antibody to N full-length protein or S full-length protein can be detected with a direct method of double-antibody sandwich, or an indirect method, wherein the antibody of the N full-length protein or the S full-length protein (in the specimen, for example in blood) is actually be deemed as an antigen. When detecting the antibody of the N or S full-length protein, the second antibody of the anti-N full-length protein antibody may be coupled to a marker substance, and a second antibody of the anti-N full-length protein antibody may be immobilized in the detection area. This is the so called double-antibody sandwich method. Of course, optionally, the marker substance is coupled to the first antibody of the anti-N full-length protein antibody, and the second antibody of the first antibody is immobilized in the detection area, that is the so-called indirect method.

In some other embodiments, if the test strip 2 is only used to prevent missing detection, the above antibody setting method may also be used; when the first antibody of the antibody to the S-RBD site protein is coated in the marker area, the second antibody of the antibody to the anti-S-RBD site protein is immobilized in the detection area, or the first and the second antibodies of the antibody to the anti-S-RBD site protein are immobilized in the detection area

Further, the detection area also has a control line. The control line is set to react with a substance in the marker area to produce a substance containing a marker compound, and generally, goat anti-chicken IgY antibodies and goat anti-mouse IgG antibodies are used.

Besides, the said first test strip or the second test strip further comprises a water absorption area for adding buffer solution.

The water absorption area can also be called a buffer solution feeding area, and the buffer solution therein may be a buffer solution PBS.

On the other hand, the present invention provides a usage of two antigen combinations for preparing a lateral flow detection device for detecting a novel coronavirus by immunoassay, wherein the first combination contains the S full-length protein antigen and/or N full-length protein antigen of the novel coronavirus; the second combination contains the S-RBD site protein antigen of the novel coronavirus; the amino acid sequence of the S full-length protein is indicated by SEQ ID NO.3, and the amino acid sequence of the N full-length protein is indicated by SEQ ID NO.2, and the amino acid sequence of the S-RBD site protein is indicated by SEQ ID NO.1.

A preparation method of the lateral flow detection device for detecting a novel coronavirus by immunoassay provided by the present invention:
(1) Detection area: use a nitrocellulose filter membrane, dissolve the antibody on the test line with a buffer solution PBS, and then use a film coating equipment to mark a line on the nitrate membrane to keep the distance between the different antibodies as 3-8 mm, and then dry the nitrocellulose filter membrane in an oven for later use.
(2) Sample area: use a sample feeding pad, which is made of glass fiber.
(3) Marker area: prepare the antigen or antibody marked by gold particles, and then spray the marked mixture on the polyester film through a spraying device to make a marker pad.
(4) Water absorption area: use an absorbent pad made of ordinary absorbent filter paper.
(5) Assembly: overlay one end of the sample feeding pad on the marker pad to superimpose the marker pad on the nitrocellulose filter membrane, and make one end of the nitrocellulose filter membrane superimposed by the absorbent filter paper, thus to form a whole test strip, and then assemble it in the test card, wherein a sample feeding hole on the test card corresponds to the sample feeding pad, and the nitrocellulose filter membrane corresponds to a degree window.

A lateral flow detection device for detecting a novel coronavirus by immunoassay provided by the present invention follows the use method below:
Take an appropriate amount of sample and add it to the sample area of the detection device, after standing for some time, determine a presence of a novel coronavirus antibody in the sample according to an indication of the test line and the control line; compare the color of the test line with the standard color card, if the color value is less than 3, the result is deemed as negative, and if it is greater than or equal to 3, the result is deemed as positive.

Compared with the prior art, the invention has the beneficiary effects that, The invention provides a lateral flow detection device for detecting a coronavirus by immunoassay, wherein the detection device comprises two lateral flow test strips, a test strip 1 is used to test an antibody to a N full-length protein and/or a S full-length protein, while a test strip 2 is used to test an antibody to a S-RBD site protein, and a combination of the two test strips is used to test IgG and IgM antibodies to novel coronavirus, which further improves a detection rate of serological antibodies and effectively reduces a possibility of missing detection and wrong detection, thereby avoiding any missing detection. Simultaneously, the device could be further used to identify an effective antibody produced by an innate immunity and/or after an patient's recovery from disease, so the two test strips are significantly complementary to each other. The detection device of the present invention has the advantages that it detects reliably and efficiently in a simple manner; it can prevent missing detection as possible, it operates conveniently without need of professional instruments and personnel, and easily popularized for clinical promotion and application. Further, the present invention provided two antigen combinations for preparing detection devices for rapid detection of a novel coronavirus and an application thereof.

### Details of Drawings

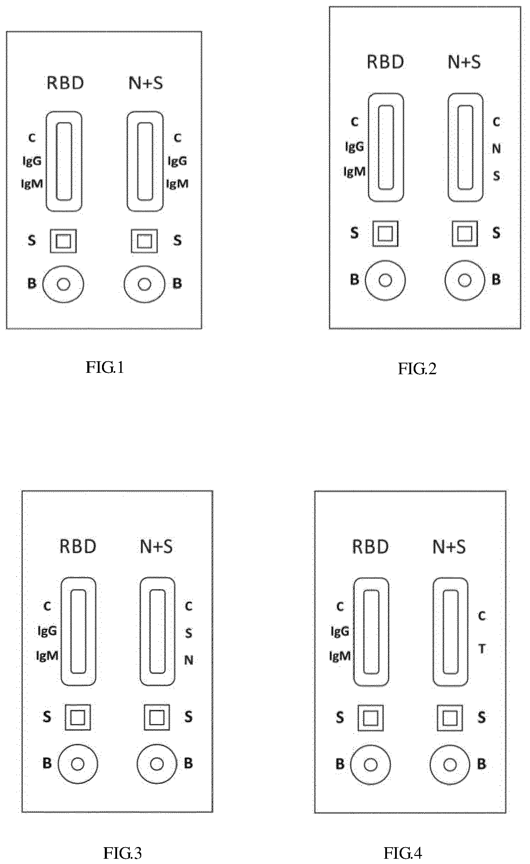

FIG.1 depicts a schematic diagram of an embodiment of the present invention, for different combinations used in detection of IgG and IgM, two test lines are provided on each test strip.
FIG.2 depicts a schematic diagram of another embodiment of the present invention, for different combinations, RBD is used for detection of IgG and IgM; while for N and S, it used to detect an antibody to N antigen or an antibody to S antigen, or N antigen or S antigen.
FIG.3 depicts a schematic diagram of another embodiment of the present invention, for different combinations, RBD is used for detecting IgG and IgM; while for N and S, it used to detect an antibody to N antigen or an antibody to S antigen, or N antigen or S antigen.
FIG.4 depicts a schematic diagram of another embodiment of the present invention, for different combinations, RBD is used for detecting IgG and IgM; while for N+S, it used to detect an antibody to N antigen or an antibody to S antigen, or N antigen or S antigen; as long as an N or S antibody, or an N/or S antigen is present in the sample, there is a test line (T).

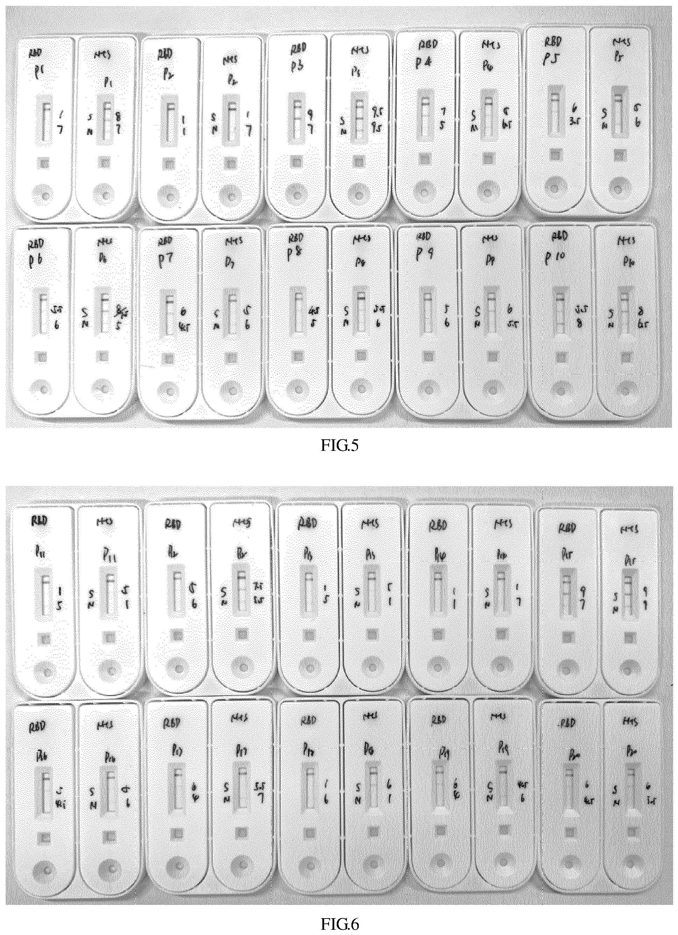

FIG.5 depicts a test result picture of embodiment 3 of the present invention, indicating the actual test result of schematic diagram shown in FIG.3 (the test results of positive samples 1-10) (when detecting the S and N antibodies, if S and N are separated on the same test strip, which indicates the S test line and the N test line are used to detect the antibody IgG and/or IgM of S antigen or an antigen in the blood).
FIG.6 depicts a test result picture of embodiment 3 of the present invention, indicating the actual test result of schematic diagram shown in FIG.3 (the test results of positive samples 11-20) (when detecting the S and N antibodies, if S and N are separated on the same test strip, which indicates the S test line and the N test line)

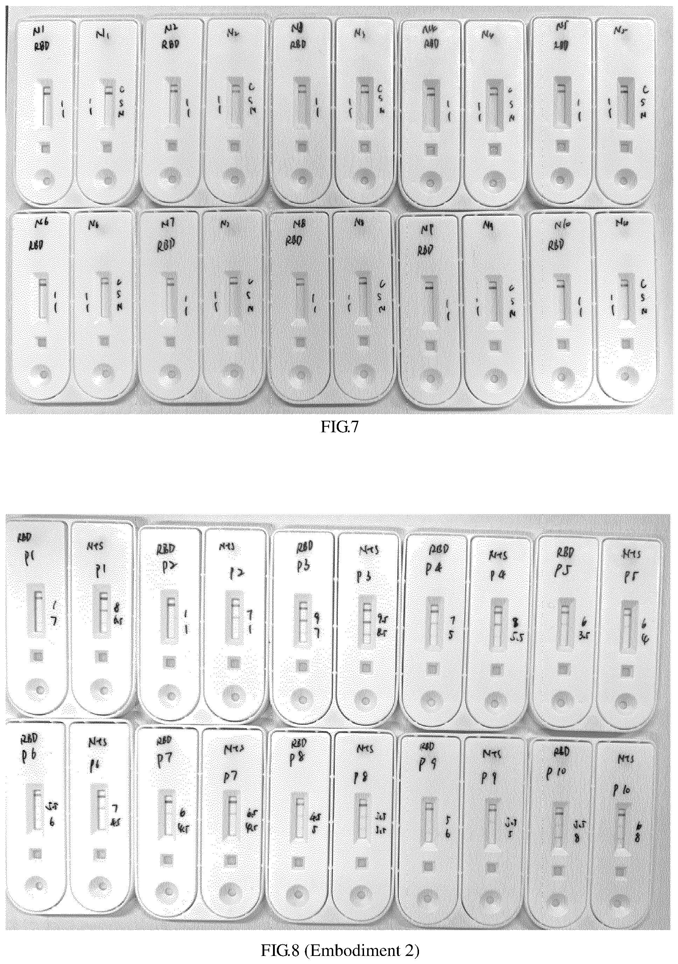

FIG.7 depicts a test result picture of embodiment 3 of the present invention, indicating the actual test result of schematic diagram shown in FIG.3 (the test results of positive samples 1-10) (when detecting the S and N antibodies, if S and N are separated on the same test strip, which indicates S test line and N test line are used to detect a presence of an antibody of the S or N antigen in a blood sample)
FIG.8 depicts a test result picture of embodiment 2 of the present invention, indicating the actual test result of schematic diagram shown in FIG.1 (the test results of positive samples 1-10) (RBD is used to detect IgM and IgG antibodies in the blood; N+S antibodies correspond to the S test line and the N test line, as long as an antibody is present in the two antigens, a positive result will be obtained).

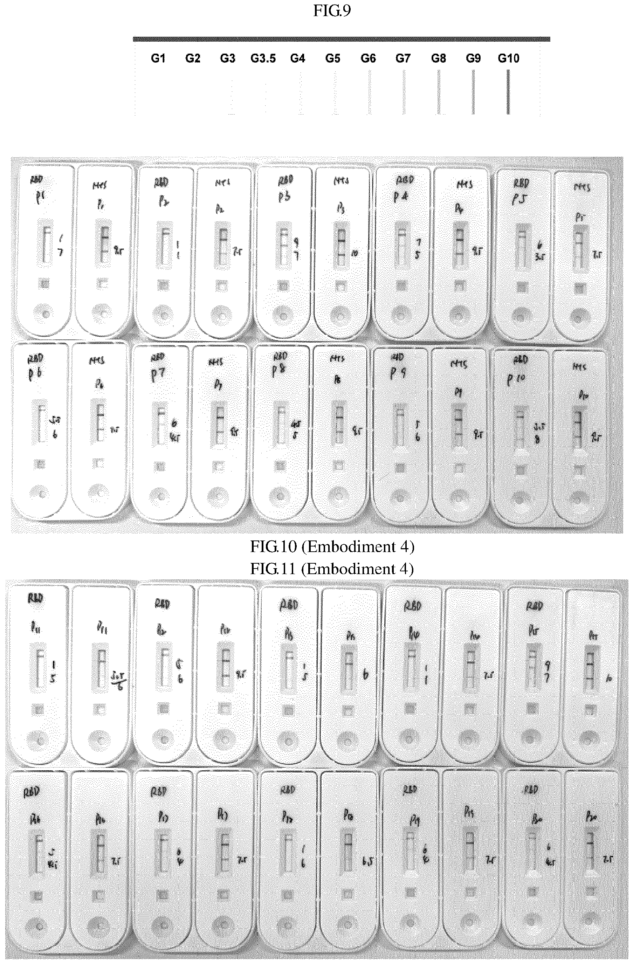

FIG9 depicts a standard color card used in the present invention for judging a color value or depth of the T line.
FIG 10 and Fig. 11 is the test results as the example 4.

### Detailed description

The following is a further explanation of the structures or of the technical terms involved in the invention, unless specifically specified, they will be understood and interpreted in accordance with the general terms in use in the field. The explanations merely take examples to illustrate how the method of the invention is realized and do not constitute any limitation on the invention. The scope of the invention is limited and expressed by the claims.

### Detection

Detection means to conduct an experiment or a test to determine the presence of a substance or material. The said substance or material, for example, but not limited to chemicals, organic compounds, inorganic compounds, metabolic products, drugs or drug metabolites, organic tissue or metabolites of organic tissues, nucleic acids, proteins, or polymers. In addition, detection can also indicate the quantity of a substance or material tested. Furthermore, a test also means immunity test, chemical test, enzyme test, etc.

### Specimen

In the present invention, the specimen used by the detection device includes a biological fluid. The said specimen can be initially liquid, solid or semi-solid. A solid or semi-solid specimen can be converted into a liquid specimen by any suitable method of mixing, crashing, macerating, incubating, dissolving and enzymatic hydrolysis, and then pour into a collecting chamber and be tested for presence of an analyte. The specimen can be taken from a human body, an animal, a plant and nature. The specimen taken from the human body, can be a liquid specimen such as blood, serum, urine, cerebrospinal fluid, sweat, lymph, saliva, gastric fluid; or a solid or semi-solid specimen such as feces, hair, keratin, tartar, nail. The specimen taken from a plant may be solid specimens such as roots, stems and leaves; and also liquid or semi-solid specimens such as tissue fluids and cell fluids prepared from roots, stems and leaves. The specimen taken from the nature may be liquid specimens such as rainwater, river water, seawater, groundwater, etc.; and also solid or semi-solid specimens such as soil, rock, ore, petroleum, etc.

In some embodiments, the specimen described in the present invention may be serum or whole blood or plasma, or when detecting an antigen, the specimen may be throat swab, nasal swab or taken from lung.

### Detection device

The detection device generally comprises a test element, wherein the so-called test element refers to a component that can detect the analyte in the specimen. The test element can test the analyte based on any technical principle, for example, immunology, chemistry, electricity, optics, molecular science, physics, etc. The test element of the present invention may be one kind or a combination of two or more kinds of test elements. The test element has a detection area for displaying a test result, and the detection area displays the test result after the detection.

Various testing elements can be combined and used in the present invention. One of the forms is a test strip. A test strip used to analyze the analyte in a specimen (such as a drug or a metabolite that indicates a medical condition), can be in various forms, such as immunoassay or chemical analysis. Test paper can adopt the analysis mode of a non-competition law or a competition law. The test paper generally includes an absorbent material with a specimen feeding area, a reagent area and a test area. The specimen is added to the specimen feeding area, and flows to the reagent area through capillary action. In the reagent area, if an analyte is present, the specimen will bind to the reagent. Then, the specimen continues to flow to the detection area. Other reagents, such as molecules specifically bonded with the analyst, are fixed in the detection area. The reagents react with the analyte (if any) in the specimen and bind to the analyte in the area or bind to one of the reagents in the reagent area. The marker used to show a detection signal exists in a reagent area or a separated marker area.

The typical non-competitive analysis model is that if the specimen includes the analyte, a signal can be generated; if the specimen does not include the analyte, a signal may not be generated. In competition law, if the analyte does not exist in the specimen, a signal may be generated; if the analyte exists, a signal may not be generated.

The testing element may be a kind of test paper, and it can also be an absorbent material or a non-absorbent material. The test paper can include various materials for transferring a liquid specimen. Wherein, the material of one kind of the test paper may be covered over another material, for example a filter paper covered over a nitrocellulose filter membrane. One area of the test paper can use one or more materials, and the other area can use one or more of the other different materials. The test paper can be attached to a support or a hard surface to improve the strength to hold the test paper.

The analyte is detected by a signal generating system, fixing one or more compositions of the signal generating system in the analyte detection area of the test paper by using one or more enzymes that react specifically with the analyte, and or the method of fixing the specific binding substance on the test paper as described above. The substance that produces a signal may be in the feeding area, the reagent area, or the detection area, or on the entire test paper, and the substance may be filled with one or more of the materials on the test paper. A solution including a signifier is added on the surface of the test paper or one or more of the materials of the test paper are immersed in a solution containing a signifier, and then the test paper containing the signifier solution is dried up.

All areas of the test paper can be arranged in the following ways: a specimen feeding area, a reagent area, a detection area, a control area, an area for determining adulteration in the specimen and a liquid specimen absorption area. The control area is located behind the detection area. All areas can be arranged on a piece of test paper containing only one material. However, different materials are used in different areas. All areas can be in direct contact with the liquid specimen, or different areas can be arranged according to the flow direction of the liquid specimen, and the rear end of each area is connected and to the front end of another area and overlapped with each other. The materials used can be excellent water-absorbent materials, such as filter paper, glass fiber or nitrocellulose filter membrane. The test paper can also be used in other forms.

The commonly used reagent strip is a nitrocellulose filter membrane reagent strip. The detection area includes a nitrocellulose filter membrane, and specific binding molecules are fixed on the nitrocellulose filter membrane to indicate the test result; it can also be a cellulose acetate membrane or a nylon membrane. For example, the test strip or device containing a test strip of the following patents: US 4857453; US 5073484; US 5119831; US 5185127; US 5275785; US 5416000; US 5504013; US 5602040; US 5622871; US 5654162; US 5656503; US 5686315;US 5766961; US 5770460; US 5916815; US 5976895; US 6248598; US 6140136;US 6187269; US 6187598; US 6228660; US 6235241; US 6306642; US 6352862; US 6372515; US 6379620; and US 6403383. The test strips disclosed in the above patent documents and the similar devices with a test strip can be applied to the testing element or testing device of the invention for detecting analyte, for example the detection of a divided substance from the specimen.

The test strips applied to the present invention can be commonly referred to as a lateral flow test strip, and the specific structure and detection principle of the test strips are known to general technicians in the field in the prior art. An ordinary test strip comprises a specimen collection area, a marker area, a detection area and a water absorption area, wherein the specimen collection area includes a specimen receiving pad, the marker area includes a marking pad, the water absorption area can include a water-absorbing pad, the detection area includes the necessary chemical substances that can detect the presence of an analyte, such as an immunological reagent or an enzyme chemical reagent. The commonly used test strip is a nitrocellulose filter membrane strip, that is, the detection area includes a nitrocellulose filter membrane, and specific binding molecules are fixed on the nitrocellulose filter membrane to indicate a test result; it can also be a cellulose acetate membrane or a nylon membrane, etc. Also, the detection area can also include a test result control area in the downstream, generally, the control area and the detection area appear in the form of horizontal lines, which are called a test line or a control line. The test strip is a conventional reagent strip, and it can also be other types of reagent strips that detect by the capillary action. Furthermore, a test strip generally includes a dry chemical reagent component, such as a fixed antibody or any other reagent, when encountering a liquid, the liquid flows along the reagent strip under the capillary action, and the dry reagent component is dissolved in the liquid during the flow process, reacts with the dry reagent of the area in the next area, thus to carry out the necessary detection. The liquid flows depending on the capillary action. These test elements are described and documented in the following documents: *Study on Regeneration Treatment and Protein Adsorption of Nitrocellulose Filter Membranes* by Li Fugang; *Analysis on Membrane Material Performance in Colloidal Gold Test Strip* by Ma Hongyan, Li Qiang, et.al; *A New Colloidal Gold Immunochromatographic Test Strip* by Wang Yong, Wang Luhai, et.al; All of the above can be applied to the detection device of the present invention, or arranged in a detection chamber to get contact with a liquid specimen, or used to detect a presence or amount of an analyte in a liquid specimen that enters the detection chamber.

The detection device therein comprises two lateral flow test strips, wherein one test strip is used to detect IgG and IgG antibodies in a blood sample of RBD or used to detect a presence of a virus antigen, such as RBD antigen, or an N protein antigen, in a throat swab, nasal swab, pulmonary fluid, or sputum, nasal mucus. The other test strip is used to detect a blood IgG and or IgG of S or N proteins; or used to detect a presence of a virus antigen, or an N protein antigen, in a throat swab, nasal swab, pulmonary fluid, or sputum, nasal mucus.

### Coronavirus

The "coronavirus" described therein includes the following viruses: SARS, MERS and COVID-19, though they have some differences in terms of epidemiology. Globally, 10% to 30% of upper respiratory infections are caused by the four types of coronaviruses, HCoV-229E, HCoV-OC43, HCoV-NL63 and HCoV-HKU1, ranking second in a cause of the common cold, second only to rhinovirus. The infection is seasonal, with a high incidence rate of diseases in spring and winter. The incubation period is 2-5 days, and people are generally susceptible, and transmission is generally density dependent as contacts.

SARS is caused by a human infection with SARS-CoV. It first occurs in partial regions of Guangdong Province in China, and then spread to 24 provinces, autonomous regions, municipalities directly under the Central Government, and 28 other countries and regions in the world. During the first prevalence of SARS in the world between November 2002 and July 2003, 8096clinically diagnosed cases were reported globally, with 774deaths and a fatality rate of 9.6%. The incubation period of SARS is generally limited to 2 weeks, about 2 to 10 days. The crowed is generally susceptible. SARS-infected patients are the main source of infection, of which the ones with obvious symptoms are more infectious and the ones who are cured or with incubation period are not infectious.

MERS is a viral respiratory disease caused by MERS-CoV, which was first identified in Saudi Arabia in 2012. Since 2012, MERS has affected a total of 27 countries and regions in the Middle East, Asia and Europe, and 80% of cases come from Saudi Arabia, with a fatality rate of about 35%. The incubation period is 14 days at most, and people are generally susceptible. A dromedary camel is a major host of MERS-CoV and a main source of infection in human cases, with limited transmission capacity between human beings.

A severe cold caused by coronavirus (COVID-19) broke out in Wuhan in 2019. Till March 10, 2020, the cumulative number of domestic infections exceeded 80,000, with a severe rate of about 18.5% and a fatality rate of about 2%. Meanwhile, COVID-19 overseas is also in the outbreak period. The high infectivity, high pathogenicity, high severity rate and high fatality rate of such a coronavirus have caused great harm to the global economy, society and health.

### Detailed description

In combination with the embodiments below, a further description of the present invention is given. It should be noted that, the following embodiments are intended to facilitate the understanding of the present invention and do not constitute any limitation on the invention. The reagents not specifically mentioned in the embodiment are known products, which are obtained by purchasing the commercially available products.

### Embodiment 1 Preparation of the lateral flow detection device for detecting a novel coronavirus by immunoassay provided by the present invention:

The lateral flow detection device for detecting a novel coronavirus by immunoassay prepared in the embodiment is shown in FIGS.1 and 5, comprising a test strip 1 and a test strip 2, wherein the test strip 1 and the test strip 2 have basically the same structure, according to a liquid flow direction, a sample area, a marker area, a detection area and a water absorption area are arranged in order from the upstream to the downstream; the sample area comprises a sample feeding hole S and a buffer solution hole B which are located on the sample area; the water absorption area uses ordinary absorbent filter paper as an absorbent pad; the sample area uses a sample feeding pad and the sample feeding pad is made of glass fiber, so that the sample added through the sample feeding hole S flows onto the glass fiber, the buffer solution added through the hole B flows onto the glass fiber and then mixes with the sample and flows onto the marker pad; the marker area is made into a marker pad, comprising the antigen or antibody coupled to marker particles (such as gold particles, latex particles or dyes, or other colored marker substances), the marker mixture is sprayed on a polyester film through a spraying equipment to form a marker pad, the marker substances on the marker pad flow with the liquid; the detection area adopts a nitrocellulose filter membrane, the antibody on the test line is dissolved with a buffer solution PBS, and then a film coating equipment is used to mark a line on the nitrate membrane to keep the distance between the different antibodies as 3-8 mm, and then the nitrocellulose filter membrane is dried in an oven for later use, and generally the antibody, antigen or other mixture treated on the membrane does not move.

After completing preparation of the water absorption area, sample area, marker area, and detection area respectively, assembly is conducted by the following steps: overlaying one end of the sample feeding pad on the marker pad to superimpose the marker pad on the nitrocellulose filter membrane, and making one end of the nitrocellulose filter membrane superimposed by the absorbent filter paper, thus to form a whole test strip, and then assembling it in the test card, wherein a sample feeding hole S and a buffer solution bole B on the test card correspond to the sample feeding pad, the nitrocellulose filter membrane corresponds to a degree window, and the feeding hole S is located on the downstream of the buffer solution bole B.

### Embodiment 2: the full-length S protein antigen and the N protein antigen are coated in the marker area of the test strip 1, and the RBD antigen is coated in the marker area of the test strip 2.

A preparation method of the lateral flow detection device for detecting a novel coronavirus by immunoassay described in the embodiment refers to the implementation method of embodiment 1, and the only difference lies in the marker pad and the reagent on the test line.

Test strip 1: On the nitrocellulose filter membrane, the control line is coated with a goat anti-mouse IgG and/or a goat anti-chicken IgY antibody, the IgG test line is coated with a mouse anti-human IgG antibody, the IgM test line is coated or immobilized with mouse anti-human IgM antibody, and the marker pad is coated with the full-length S protein*GOLD and the full-length N protein (antigen) antibody, and anti-chicken IgY antibody*GOLD (for control line). When treating the marks, the mass ratio of the S full-length protein to the N full-length protein is 20:1-5:1. Therein, the mass ratio of the S full-length protein to the N full-length protein is 20:1 (the test strip on the right of the diagram in FIG.1).

Test strip 2: on the nitrocellulose filter membrane, the control line is coated or immobilized with a goat anti-mouse IgG and/or a goat anti-chicken IgY antibody, the IgG test line is coated with a mouse anti-human IgG antibody, the IgM test line is coated with mouse anti-human IgM antibody, and the marker pad is coated with RBD antigen *GOLD and the anti chicken IgY antibody*GOLD. The IgG test line is used to detect IgG in a specimen, and the IgM test line is used to detect IgM in a specimen; and they will be treated in accordance with the implement ion method in the detection area described in embodiment 1 (the test strip on the left in the diagram of FIG.1).

The detection principle of the test strip 1 is: if an antibody (IgG or IgM) to S protein or N full-length protein (antigen) presents in the blood specimen, the antigen on the marker pad will bind to the antibody in the specimen to form: metal particles-S protein or N full-length protein-IgG or IgM (specimen), and then the granular complex is captured by the anti human IgG antibody immobilized on the test line, or anti human IgM antibody, thus to produce a positive or negative result (metal particles -S protein or N full-length protein (antigen) - IgG or IgM (specimen) - anti human IgG antibody, or anti human IgM antibody) (indirect method). AN or S protein may be detected if one or both of them produces an antibody due to an infection in a human body.

The detection principle of the test strip 2 is: if an antibody (IgG or IgM) to S protein-RBD (identification domain) presents in the blood specimen, the antigen on the marker pad will bind to the antibody in the specimen to form: metal particles-S protein-RBD-IgG or IgM, and then the granular complex is captured by the anti human IgG antibody immobilized on the test line, or anti human IgM antibody, thus to produce a positive or negative result (metal particles -S protein-RBD (antigen) - IgG or IgM (specimen) - anti human IgG antibody, or anti human IgM antibody).

The test strip 1 and the test strip 2 are respectively placed in a test card, a positive blood sample or a negative blood sample is added to the sample feeding hole S (a square hole), and buffer solution is added to another feeding hole B (a round hole): the composition of the buffer solution is phosphate buffer, with pH=7.4. After testing 20 positive samples P1-P20 (these positive samples are clinically confirmed positive samples: the samples are detected as containing a coronavirus after receiving a nucleic acid testing of a throat swab sample) and 20 negative samples N1-N20 (the clinically confirmed samples-the samples are negative samples as they are detected as containing a coronavirus after receiving a nucleic acid testing of a throat swab sample), the results obtained are shown in Table 1:

**Table 1: Test results of embodiment 2**

| No. | (N+S) (reading) | | (RBD) (reading) | |
|---|---|---|---|---|
| | IgG | IgM | IgG | IgM |
| P1 | 8 | 6.5 | 1 | 7 |
| P2 | 7 | 1 | 1 | 1 |
| P3 | 9.5 | 8.5 | 9 | 7 |
| P4 | 8 | 5.5 | 7 | 5 |
| P5 | 6 | 3.5 | 6 | 3.5 |
| P6 | 7 | 4.5 | 5.5 | 6 |
| P7 | 6 | 4.5 | 6 | 4.5 |
| P8 | 4.5 | 5 | 4.5 | 5 |
| P9 | 5 | 6 | 5 | 6 |
| P10 | 6 | 8 | 5.5 | 8.5 |
| P11 | 1 | 5 | 1 | 5 |
| P12 | 7 | 5 | 5 | 6 |
| P13 | 1 | 5.5 | 1 | 5 |
| P14 | 7 | 1 | 4 | 1 |
| P15 | 9 | 8 | 9 | 7 |
| P16 | 6 | 4 | 5 | 4.5 |
| P17 | 7 | 5 | 6 | 4 |
| P18 | 1 | 6 | 1 | 6 |
| P19 | 6 | 4.5 | 6 | 4 |
| P20 | 6 | 4.5 | 6 | 4.5 |
| N1 | 1 | 1 | 1 | 1 |
| N2 | 1 | 1 | 1 | 1 |
| N3 | 1 | 1 | 1 | 1 |
| N4 | 1 | 1 | 1 | 1 |
| N5 | 1 | 1 | 1 | 1 |
| N6 | 1 | 1 | 1 | 1 |
| N7 | 1 | 1 | 1 | 1 |
| N8 | 1 | 1 | 1 | 1 |
| N9 | 1 | 1 | 1 | 1 |
| N10 | 1 | 1 | 1 | 1 |
| N11 | 1 | 1 | 1 | 1 |
| N12 | 1 | 1 | 1 | 1 |
| N13 | 1 | 1 | 1 | 1 |
| N14 | 1 | 1 | 1 | 1 |
| N15 | 1 | 1 | 1 | 1 |
| N16 | 1 | 1 | 1 | 1 |
| N17 | 1 | 1 | 1 | 1 |
| N18 | 1 | 1 | 1 | 1 |
| N19 | 1 | 1 | 1 | 1 |
| N20 | 1 | 1 | 1 | 1 |

Comparing the color value or depth of the test line with a standard color card (FIG.9) (the color card is also a concentration of a positive structure line at different concentrations, forming a gradient due to different color depths from G1 to G10), if the color value of the test line is less than 3 (G3), it is judged as negative, and if the value is greater than or equal to 3 (G3), it is judged as positive. See partial positive results in FIG. 8 for the specific results, with the negative results not shown.

As shown in Table 1, for negative samples N1-N20, the readings on the test strip 1 and the test strip 2 are both 1, indicating that they are both negative, which are consistent with a result of actual samples and a result obtained from a nucleic acid testing. For positive samples P1-P20, if the detection is only conducted for the RBD antigens marked with color particles (the test strip 2), for IgG, there are 5 negative results (P1, P2, P11, P13 and P18) in the 5 samples that are confirmed as positive; however in the test result of N+S (test strip 1), for IgG, there are 3 negative results (P11, P13 and P18) in the 5 positive samples, and the other samples are positive (P1, P2). It indicates that at least 2 more samples are detected as positive by N+S testing. IgG is an antibody developed after infection or after recovery, it indicates that only detecting IgG antibody produced by RBD may cause the missing detection. The case may be that samples that are positive in nucleic acid testing are throat swab samples, while the present invention uses a blood sample. Generally, an antibody produced by blood is only developed a few days after infection, for example, IgM is developed at first and followed by IgG. If only an RMD antigen is used to detect antibodies, it may result in missing IgG detection, then it may be considered that although these samples are positive for IgM but negative for IgG, and this will lead to inappropriate or wrong therapeutic measures.

In combination with the results of IgM below, for P2 samples, when an RBD antigen is used for antibody detection, the results show both IgM and IgG are negative, then the patient may be considered negative and no further nucleic acid testing will be performed for confirmation, which may cause missing detection, thereby leading to a wider range of contagion, or infecting those healthy people. On the contrary, if N+S detection is adopted as a supplementary testing, the P2 sample will be positive, and at least IgG is detected as positive; although IgG is deemed as a sign of a patient's recovery or previous infection, to the minimum, it could promote change in a treatment strategy or a medical treatment or protection measures, for example, conducting a nucleic acid testing to detect a presence of a virus in the body.

For P1 samples, both IgM and IgG are positive in N+S combined testing; if RBD alone is used for detection, then IgM is positive and IgG is negative, this indicates that RBD detection alone is not comprehensive, so no comprehensive assessment can be made. N+S detection can comprehensively evaluate a patient's infection status.

For IgM, the RBD antigen and the N+S antigen have the same results after detection: both of them are negative (P2 and P14), but the readings detected are different, some samples obtain higher degrees (P3, P4, P13, P15, etc.) when the test strip 1 is used; and some samples obtain higher degrees (such as P1, P6, P10, P16, etc.) when the test strip 2 is used. This shows that, in some samples, N+S has a high overall antibody concentration in the blood, while the concentration of RBD antibody is low, or, for some samples, the concentration of the RBD antibody is high while the concentration of the N+S antibody is low. At least, it shows that, when some samples are in an uncertain state, for example, its reading on the color card is between 3.0-3.5, if a single indicator is used, for example, only RBD, or only N or S is used for detection, then the result is uncertain. However, if a combined testing is used, from their different test results, reliability of the test results can be further verified, to avoid missing detection, or avoid false negatives or false positives, especially false negatives.

For example, RBD detection is used for a sample similar to P17, the results are 6 (IgG) and 4 (IgM), while if N+S is used, the results are 7 (IgG) and 5 (IgM), there is just a difference of a color level. For similar samples, an antibody content of the sample or the binding ability of the antibody to an antigen is lower than that of the P17 sample; when RBD detection is used, the results will be 5 (IgG) and 3 (IgM), at this time, IgM is considered as negative. However, when N+S is used as a supplementary detection, the result may be 5 (IgG) and 4 (IgM), and IgM is considered as positive. For a more extreme example, when RBD detection is used alone, the results are 2 (IgG) and 2 (IgM), and the sample is considered as negative; but if the N+S supplementary detection is used, the results are 3 (IgG)) and 3 (IgM), it may be judged that the patient where the sample is taken from is infected with a virus or is likely to have infected with a virus, at least, it could remind the tester that the patient of the sample needs further detection for confirmation, for example nucleic acid confirmation testing.

This is because, in terms of the infection and transmission route of a novel coronavirus, the patient may be inclined to have been infected with the novel coronavirus and is now in an acute infection period, it is necessary to make further confirmation or isolation treatment to confirm the originally negative result as more positive. Of course, at this time, a nucleic acid testing of a throat swab or a blood sample could be conducted for further confirmation. After all, some virus carriers do not show symptoms themselves, but they are contagious. In this way, it is possible to get more accurate test results, so that appropriate measures could be adopted for further treatment and protective measures could be taken.

To sum up the above, when testing IgG and IgM at the same time, the test strip 2 will cause missing detection of P2 and P14; if the test strip 1 is used for supplementary testing, missing detection may be completely avoided; for detection of IgG, if both the test strip 1 and the test strip 2 are used for detection, it is conductive to avoid missing detection of IgG antibodies, increase the comprehensiveness of the detection and prevent missing detection; for detection of IgM, although in the test result, both the test strips 1 and 2 detect IgM as positive, and the anti-missing detection effect is not good, but it cannot be denied that the test strip 1 and the test strip 2 are complementary to each other; if there is a weak positive sample (when the IgM antibody content is relatively low), a complementary detection is made based on a difference between the readings of the test strip 1 and the test strip 2, which may exert an obvious anti-missing detection effect. If the RBD antibody is detected, the probability of a negative result may be very high; if N+S is detected as supplementary, a positive result may be obtained, which will be a make-up for a natural defect of the RBD antibody detection (RBD shows weak positive in some samples).

A large number of experiments have been made in this respect, when RBD detection is used alone, we randomly selected 50 blood samples for testing, of which 5 samples show weak positive (both for IgG and IgM), and the reading is about 3 or close to 3; while when N+S detection is used, they all show positive, and the reading is between 4-5. The patients of these 5 samples are tested by nucleic acid testing, with samples as throat swabs; the results show all the samples are confirmed as positive. This further confirms that single detection and combined detection are suitable for useful and supplementary confirmation.

### Embodiment 3 the detection area of the test strip 1 is coated with full-length S protein and N protein, and the marker area of the test strip 2 is coated with RBD antigen.

A preparation method of the lateral flow detection device for detecting a novel coronavirus by immunoassay described in the embodiment refers to the implementation method of embodiment 1. For the test strip 1: on the nitrocellulose filter membrane, the control line is coated or immobilized with a goat anti-mouse IgG antibody, the N test line is coated with a full-length N protein (antigen), and the S test line is coated with a full-length S protein (antigen), the marker pad is coated with mouse anti-human IgG antibody *GOLD and mouse anti-human IgM antibody *GOLD (excessive). Wherein the N test line of the test strip 1 detects an antibody to the full-length N protein antigen (including IgG and IgM), and the S test line detects an antibody to the full-length S protein antigen (including IgG and IgM). That is to say, for N or S antigens, as long as the sample contains IgG or IgM to N antigens, or IgG or IgM for S antigens, a positive result will be shown on the N test line, otherwise a negative result will be shown.

Test strip 2: on the nitrocellulose filter membrane, the control line is coated with a goat anti-mouse IgG and/or a goat anti-chicken IgY antibody, the IgG test line is coated with a mouse anti-human IgG antibody, the IgM test line is coated with mouse anti-human IgM antibody, and the marker pad is coated with RBD antigen *GOLD and the anti chicken IgY antibody*GOLD. The IgG test line of the test strip 2 is used to detect IgG, and the IgM test line is used to detect IgM.

The detection principle of the test strip 1 is: if an antibody (IgG or IgM) to S protein or N full-length protein (antigen) presents in a blood specimen, the antigen on the marker pad will bind to the antibody in the specimen to form: metal particles-mouse anti-human IgG antibody or mouse anti-human IgM antibody (specimen), and then the granular complex is captured by a full-length N protein or a full-length S protein immobilized on the test line, or anti human IgM antibody, thus to produce a positive or negative result (metal particles -mouse anti-human IgG antibody or mouse anti-human IgM antibody - IgG or IgM (specimen) -S protein or N full-length protein).

The detection principle of the test strip 2 is: if an antibody (IgG or IgM) to S protein-RBD presents in a blood specimen, the antigen on the marker pad will bind to the antibody in the specimen to form: metal particles-S protein-RBD-IgG or IgM, and then the granular complex is captured by the anti human IgG antibody immobilized on the test line, or anti human IgM antibody, thus to produce a positive or negative result (metal particles -S protein-RBD (antigen) - IgG or IgM (specimen) - anti human IgG antibody, or anti human IgM antibody).

The test strip 1 and the test strip 2 are respectively placed in a test card, a positive blood sample is added to the sample feeding hole, and buffer solution is added to another hole: the composition of the buffer solution is phosphate buffer, with pH=7.4. After testing 20 positive samples P1-P20 (clinically confirmed positive samples) and 20 negative samples N1-N20 (clinically confirmed samples-the samples), the results obtained are shown in Table 2:

**Table 2: test results of embodiment 3**

| No. | (N+S) | | (RBD) | |
|---|---|---|---|---|
| | N | S | IgG | IgM |
| P1 | 7 | 8 | 1 | 7 |
| P2 | 7 | 1 | 1 | 1 |
| P3 | 10 | 9 | 9 | 7 |
| P4 | 7 | 5 | 7 | 5 |
| P5 | 6 | 5 | 6 | 3.5 |
| P6 | 5 | 8 | 5.5 | 6 |
| P7 | 6 | 5 | 6 | 4.5 |
| P8 | 6.5 | 5.5 | 4.5 | 5 |
| P9 | 5 | 5.5 | 5 | 6 |
| P10 | 6.5 | 8 | 5.5 | 8.5 |
| P11 | 1 | 5 | 1 | 5 |
| P12 | 5 | 8 | 5 | 6 |
| P13 | 1 | 5 | 1 | 5 |
| P14 | 7 | 1 | 4 | 1 |
| P15 | 9.5 | 9 | 9 | 7 |
| P16 | 6 | 5 | 5 | 4.5 |
| P17 | 7 | 5.5 | 6 | 4 |
| P18 | 1 | 6 | 1 | 6 |
| P19 | 6 | 4.5 | 6 | 4 |
| P20 | 5.5 | 6 | 6 | 4.5 |
| N1 | 1 | 1 | 1 | 1 |
| N2 | 1 | 1 | 1 | 1 |
| N3 | 1 | 1 | 1 | 1 |
| N4 | 1 | 1 | 1 | 1 |
| N5 | 1 | 1 | 1 | 1 |
| N6 | 1 | 1 | 1 | 1 |
| N7 | 1 | 1 | 1 | 1 |
| N8 | 1 | 1 | 1 | 1 |
| N9 | 1 | 1 | 1 | 1 |
| N10 | 1 | 1 | 1 | 1 |
| N11 | 1 | 1 | 1 | 1 |
| N12 | 1 | 1 | 1 | 1 |
| N13 | 1 | 1 | 1 | 1 |
| N14 | 1 | 1 | 1 | 1 |
| N15 | 1 | 1 | 1 | 1 |
| N16 | 1 | 1 | 1 | 1 |
| N17 | 1 | 1 | 1 | 1 |
| N18 | 1 | 1 | 1 | 1 |
| N19 | 1 | 1 | 1 | 1 |
| N20 | 1 | 1 | 1 | 1 |

Wherein, comparing the color of the test line with a standard color card; if the color value is less than 3 (G3), it is judged as negative, and if the value is greater than or equal to 3 (G3), it is judged as positive.

As shown in Table 2, for negative samples N1-N20, the readings on the test strip 1 and the test strip 2 are both 1, indicating that they are both negative, which are consistent with a result of actual samples.

For positive samples P1-P20, the test strip 2 will cause missing detection of P2, and the N protein antigen in the test strip 1 can be supplemented to prevent missing detection; while if the N protein antibody is detected separately, it may lead to missing detection of 3 samples (P11, P13, P19); at this time, detection of IgM by the S protein antibody or the RBD antibody of the test strip 2 could make supplement to prevent missing detection; if the S protein antibody is detected separately, it may lead to missing detection of 2 samples (P2, P14), detection of IgG by the N protein antibody or test strip 2 RBD antibody could make supplement to prevent missing detection. It can be seen that, the N protein and S protein of the test strip 1, as well as the RBD protein of the test strip 2 are complementary, so it can effectively reduce the possibility of missing detection, which is in compliance with the actual situation. That is to say, when an RBD antigen is used for antibody detection, missing detection of sample P2 may be caused, while the N+S combination method is used for detection, although some samples may be tested as negative by N detection alone, or some samples may be tested as negative by S detection alone. But when N+S (not distinguishing the respective detection rates of N and S separately) combined detection is used, the positive detection rate is 100%, which is consistent with the actual positive result. Generally, nucleic acid testing is a gold standard for novel coronavirus testing, as long as a blood sample or throat swab sample contains such novel coronavirus, it can be confirmed by nucleic acid testing. And through antibody detection, antibodies generally appear within a certain period of time after infection, for example, an IgM antibody appears in 5-7 days first, and then an IgG antibody appears in 10-15 days. The production of antibodies has a hysteretic nature. If a sample is collected more than 5 days later, the result of antibody detection may be negative; if only the RBD detection is used, missing detection may not be avoided anyway, and if the N+S combined detection is used, the result is 100% consistent with that of the nucleic acid testing.

### Embodiment 4:

The marker area of the test strip 2 is coated with RBD antigen, the detection area in the test strip 1 has only one test line where antibodies to N and S full-length protein antigens are immobilized. The different from the specific treatment from the test strip 1 in embodiment 3 is that N and S are not distinguished, and N and S antigens are mixed and treated in the marker area, and the same sample is used for detection, the test results are given as below.

**Table 3: Test results of Embodiment 4**

| No. | (RBD) | | (N+S) |
|---|---|---|---|
| | IgG | IgM | T |
| P1 | 1 | 7 | 9.5 |
| P2 | 1 | 1 | 7.5 |
| P3 | 9 | 7 | 10 |
| P4 | 7 | 5 | 9.5 |
| P5 | 6 | 3.5 | 7.5 |
| P6 | 5.5 | 6 | 9.5 |
| P7 | 6 | 4.5 | 9.5 |
| P8 | 4.5 | 5 | 9.5 |
| P9 | 5 | 6 | 9.5 |
| P10 | 5.5 | 8.5 | 9.5 |
| P11 | 1 | 5 | 6 |
| P12 | 5 | 6 | 9.5 |
| P13 | 1 | 5 | 6 |
| P14 | 1 | 1 | 7.5 |
| P15 | 9 | 7 | 10 |
| P16 | 5 | 4.5 | 7.5 |
| P17 | 6 | 4 | 7.5 |
| P18 | 1 | 6 | 6.5 |
| P19 | 6 | 4 | 7.5 |
| P20 | 6 | 4.5 | 7.5 |
| N1 | 1 | 1 | 1 |
| N2 | 1 | 1 | 1 |
| N3 | 1 | 1 | 1 |
| N4 | 1 | 1 | 1 |
| N5 | 1 | 1 | 1 |
| N6 | 1 | 1 | 1 |
| N7 | 1 | 1 | 1 |
| N8 | 1 | 1 | 1 |
| N9 | 1 | 1 | 1 |
| N10 | 1 | 1 | 1 |
| N11 | 1 | 1 | 1 |
| N12 | 1 | 1 | 1 |
| N13 | 1 | 1 | 1 |
| N14 | 1 | 1 | 1 |
| N15 | 1 | 1 | 1 |
| N16 | 1 | 1 | 1 |
| N17 | 1 | 1 | 1 |
| N18 | 1 | 1 | 1 |
| N19 | 1 | 1 | 1 |
| N20 | 1 | 1 | 1 |

As can be judged from the test results shown in the Table above, if the results are positive, a 100% positive result is obtained through the N+S detection method; and if only the RBD detection is used, it is not possible to get a 100% detection rate, so missing detection may be caused. This seems to indicate that, the results obtained by N+S detection are highly consistent with that obtained by nucleic acid testing, and the consistency is 100%. From another aspect, when an antibody to the N+S combined or mixed antigen is used as a test line, it can effectively detect whether a patient is infected with a coronavirus, while if only the RBD antigen is used to detect antibodies in the blood, missing detection may be caused, for example, the sample P2 is detected as positive by the nucleic acid testing, while the RBD detection shows that both IgG and IgM are negative. However, if the N+S detection is used, the result is positive, and it is strong positive (the value is 7.5), which means that the combination of RBD and N+S detections could overcome the natural defect of RBD.

### Embodiment 5: Use antibodies to detect antigens in a throat swab sample.

The marker pad on the test strip 1: it is treated with a first monoclonal antibody (AB-RBD) to anti RBD antigen*GOLD, and the AB-RBD antibody of the monoclonal antibody is immobilized on the test line. When **the throat swab sample** contains RBD antigen, the first monoclonal antibody binds to the RBD antigen to form a-AB-RBD-RBD compound substance*GOLD; when the compound substance moves to the anti-AB-RBD antibody area on the nitrocellulose filter membrane, the anti-AB-RBD antibody captures the -AB-RBD-RBD compound substance *GOLD, thereby developing color lines. Of course, a second monoclonal antibody to the RBD antigen can be immobilized on the test line (double-antibody sandwich method). According to the same principle, S antigen or S+N antigen can be detected. When detecting N+S antigens, the first monoclonal antibody (AB-S) to the anti-S antigen*GOLD and the first monoclonal antibody (AB-N) to the anti-N antigen *GOLD, the two marks are mixed together or respectively labeled to be sprayed on the marker pad respectively. A second monoclonal antibody to the S antigen and a second monoclonal antibody to the N antigen are on the test line. As long as the sample contains N or S antigen fragments, a positive result will be indicated on the test line. After testing 10 blood samples taken from patients who are clinically confirmed as being infected with the novel coronavirus, the test results are obtained as below.

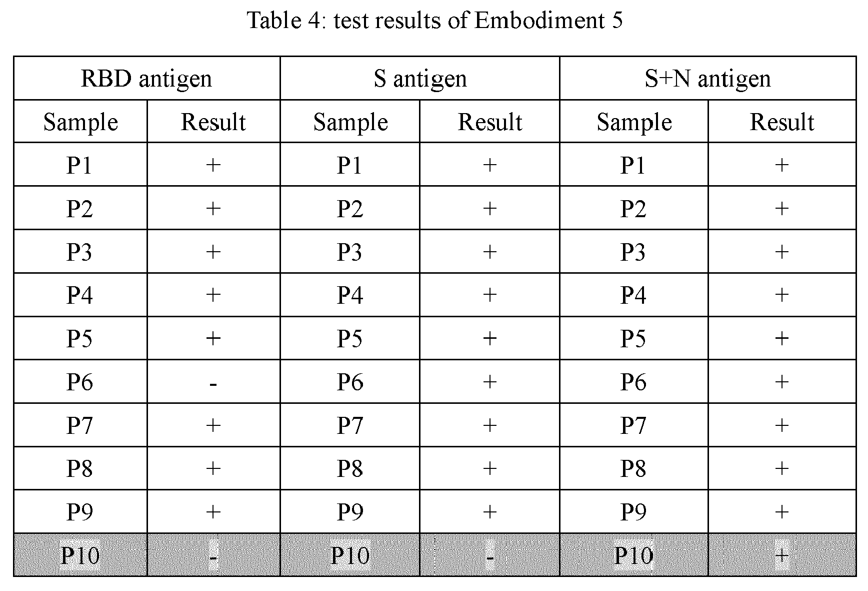

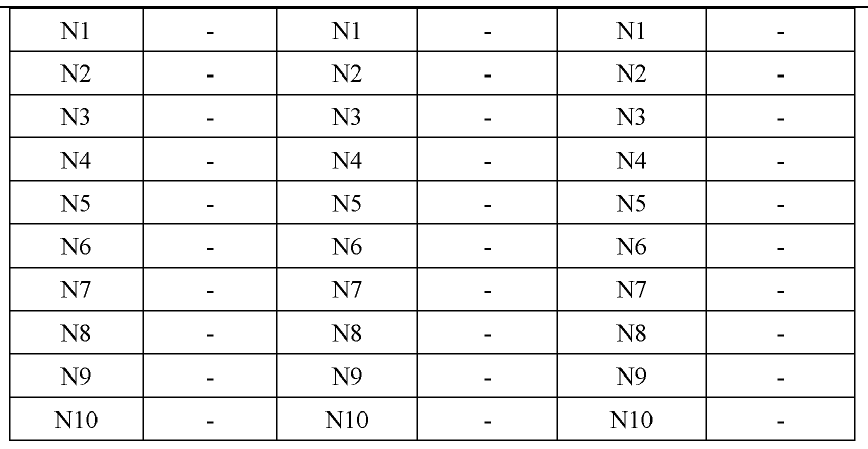

As seen from the above test results, 10 positive samples are not effectively detected by using RBD or S full-length antigens, which results in missing detection of a sample (the missing detection rate is almost 10%); and N antigen detection or N antigen + S antigen combined detection is used, and missing detection is avoided. From another aspect, if N antigen detection is used alone, a 100% detection rate may also be obtained. This seems to indicate that, considering the positive detection rate, N full-length antigen detection alone is more effective than using RBD detection alone. However, in actual detections, more detections are conducted for RBD antibodies or antigens, the reason mainly lies in that RBD is considered as the main infection area causing infection, when RBD binds to the ACE2 domain of cells, the cells may be infected; however, it ignores detection of N or S full-length sequence, full-length sequence detection can detect more sites which may cause an immune response and irritate the body to produce antibodies; generally RBD is used as a key site, but simultaneously sites of other proteins are detected separately, so as to effective avoid missing detection, increasing the detection rate, thereby effectively controlling a risk of infection.

### Embodiment 6: The detection effects of blood samples in different periods.

Test strip 1: The test strip is the same as a test strip described in embodiment 2, and it obtains different test results for a blood sample taken in different time. On the nitrocellulose filter membrane, a control line is coated with goat anti-mouse IgG or goat anti-chicken IgY antibody, an IgG test line is coated with mouse anti human IgG antibody, an IgM test line is coated with mouse anti human IgM antibody, and the marker pad is coated with antibodies of S protein-RBD *GOLD (only including the RBD antigen) and anti-chicken IgY*GOLD (control line). The detection principle is: if a S protein-RBD antibody (IgG or I gM) presents in the blood specimen, the antigen on the marker pad will bind to the antibody in the specimen to form: S protein-RBD-IgG or I gM-metal particles, and then the granular complex is captured by the immobilized anti human IgG antibody, or anti human IgM antibody, thus to produce a positive or negative result.

**Table 5: Test result of embodiment 6 (test strip 1)**

| Clinical specimens | IgM test result (positive / number of specimens) | IgG test result (positive / number of specimens) |
|---|---|---|
| 10 early clinical serum specimens (confirmed as positive by nucleic acid testing) | 6 positive/10 (positive) | 4 positive/10 (positive) |
| 10 interim clinical serum specimens (confirmed as positive) | 9 positive/10 (positive) | 9 positive/10 (positive) |
| 10 late clinical serum specimens (confirmed as positive) | 7 positive/10 (positive) | 9 positive/10 (positive) |
| 10 exceptional case specimens (confirmed as negative) | 10 negative/10 (negative) | 10 negative/10 (negative) |
| 10 whole blood specimens (confirmed as negative) | 10 negative/10 (negative) | 10 negative/10 (negative) |

If only S-RBD site antibodies are detected, for 10 positive specimen (throat swab confirmed by nucleic acid testing), there may be missing detection in the early or middle stage, and the missing detection may be serious at earlier period.

Test strip 2: The test strip is the same as a test strip described in embodiment 2, and it obtains different test results for a blood sample taken at different time. On the nitrocellulose filter membrane, the control line is coated with a goat anti-mouse IgG and/or a goat anti-chicken IgY antibody, the IgG test line is coated with a mouse anti-human IgG antibody, the IgM test line is coated with mouse anti-human IgM antibody, and the marker pad is coated with the full-length S protein*GOLD and the full-length N protein (antigen) antibody, anti chicken IgY antibody*GOLD.

**Table 6: Test result of embodiment 6 (test strip 2)**

| Clinical specimens | IgM test result (positive / number of specimens) | IgG test result (positive / number of specimens) |
|---|---|---|
| 10 early clinical serum specimens (confirmed as positive) | 6 positive/10 (positive) | 4 positive/10 (positive) |
| 10 interim clinical serum specimens (confirmed as positive) | 8 positive/10 (positive) | 10 positive/10 (positive) |
| 10 late clinical serum specimens (confirmed as positive) | 7 positive/10 (positive) | 10 positive/10 (positive) |
| 10 exceptional case specimens (confirmed as negative) | 10 negative/10 (negative) | 10 negative/10 (negative) |
| 10 whole blood specimens (confirmed as negative) | 10 negative/10 (negative) | 10 negative/10 (negative) |

The detection principle is: if a S protein-RBD antibody (IgG or I gM) presents in the blood specimen, the antigen on the marker pad will bind to the antibody in the specimen to form: metal particles-S protein or N full-length protein-IgG or I gM (specimen), and then the granular complex is captured by the immobilized anti human IgG antibody, or anti human IgM antibody, thus to produce a positive or negative result (metal particles -S protein or N full length protein (antigen) - IgG or IgM (specimen) - anti human IgG antibody, or anti human IgM antibody).

From the above results, it can be seen that the detection of full-length S and N proteins is more realistic than just detecting S-RBD proteins, and this reduces the possibility of missing detection.

Test strip 3: the test strip is the same as a test strip described in embodiment 2, and it obtains different test results for a blood sample taken at different time. On the nitrocellulose filter membrane, the control line is coated with a goat anti-mouse IgG, the S test line is coated with a full-length S protein, the N test line is coated with a full-length N protein, and the marker pad is coated with mouse anti human IgG*GOLD and mouse anti human IgM*GOLD antibodies.

**Table 7: Test result of embodiment 6 (test strip 3)**

| Clinical specimens | S test result (positive / number of specimens) | N test result (positive / number of specimens) |
|---|---|---|
| 10 early clinical serum specimens (confirmed as negative) | 6 positive/10 (positive) | 1 positive/10 (positive) |
| 10 interim clinical serum specimens (confirmed as negative) | 8 positive/10 (positive) | 10 positive/10 (positive) |
| 10 late clinical serum specimens (confirmed as negative) | 7positive/10 (positive) | 10 positive/10 (positive) |
| 10 exceptional case specimens (confirmed as negative) | 10 negative/10 (negative) | 10 negative/10 (negative) |
| 10 whole blood specimens (confirmed as negative) | 10 negative/10 (negative) | 10 negative/10 (negative) |

It can be explained from the above that the detection of N full-length sequence can be a supplementary or combined detection of S-RBD full-length sequence, which reduces the missing detection rate.

The detection principle is: if a S protein-RBD antibody (IgG or I gM) presents in the blood specimen, the mouse anti human IgG and mouse anti human IgM antibodies on the marker pad will bind to the antibody in the specimen to form: metal particles-mouse anti human IgG and mouse anti human IgM antibodies (*GOLD) -IgG or I gM (specimen), and then the granular complex is captured by the immobilized anti human N protein, thus to produce a positive or negative result (metal particles -mouse anti human IgG and mouse anti human IgM antibodies (*GOLD) - IgG or IgM (specimen) - N protein).

Test strip 4: on the nitrocellulose filter membrane, the control line is coated with a goat anti-mouse IgG and/or a goat anti-chicken IgY antibody, the S test line is coated with a full-length S protein, the N test line is coated with full-length N protein, and the marker pad is coated with the full-length S protein*GOLD, and the full-length N protein, and anti-chicken IgY antibody*GOLD.

**Table 8: Test result of embodiment 6 (test strip 4)**

| Clinical specimens | S test result (positive / number of specimens) | N test result (positive / number of specimens) |
|---|---|---|
| 10 early clinical serum specimens (confirmed as positive) | 6 positive/10 (positive) | 1 positive/10 (positive) |
| 10 interim clinical serum specimens (confirmed as positive) | 8 positive/10 (positive) | 10 positive/10 (positive) |
| 10 late clinical serum specimens (confirmed as positive) | 7 positive/10 (positive) | 10 positive/10 (positive) |
| 10 exceptional case specimens (confirmed as negative) | 10 negative/10 (negative) | 10 negative/10 (negative) |
| 10 whole blood specimens (confirmed as negative) | 10 negative/10 (negative) | 10 negative/10 (negative) |

The detection principle is: if a S protein-RBD antibody (IgG or I gM) presents in the blood specimen, the antibody will bind to the antibody in the specimen to form: a marker substance-S protein or N protein-antibody (in the specimen); it is captured by the S protein or N protein respectively immobilized in the detection area to form: a marker substance-S protein or N protein-antibody (in the specimen)-S protein or N protein.

Test strip 5: on the nitrocellulose filter membrane, the control line is coated with a goat anti-mouse IgG and/or a goat anti-chicken IgY antibody, the test line (T) is coated with full-length S protein (antigen) and full-length N protein (antigen), and the marker pad is coated with the antibodies of full-length S protein*GOLD, the full-length N protein and antibody and anti-chicken IgY*GOLD.

**Table 9: Test result of embodiment 6 (test strip 5)**

| Clinical specimens | Test result (positive / number of specimens) |
|---|---|
| 10 early clinical serum specimens (confirmed as positive) | 6 positive/10 (positive) |
| 10 interim clinical serum specimens (confirmed as positive) | 10 positive/10 (positive) |
| 10 late clinical serum specimens (confirmed as positive) | 10 positive/10 (positive) |
| 10 exceptional case specimens (confirmed as negative) | 10 negative/10 (negative) |
| 10 whole blood specimens (confirmed as negative) | 10 negative/10 (negative) |

Simultaneous detection of S and N proteins reduces the possibility of missing detection in just detecting S-RBD proteins, it makes the test result closer to the true.

Test strip 6: on the nitrocellulose filter membrane, the control line is coated with a goat anti-mouse IgG and/or a goat anti-chicken IgY antibody, the IgG test line is coated with a mouse anti-human IgG antibody, the IgM test line is coated with mouse anti-human IgM antibody, and the marker pad is coated with the full-length S protein*GOLD and the full-length N protein (antigen) antibody, anti chicken IgY antibody*GOLD.

**Table 10: Test result of embodiment 6 (test strip 6)**

| Clinical specimens | Test result (positive / number of specimens) |
|---|---|
| 10 early clinical serum specimens (confirmed as positive) | 6 positive/10 (positive) |
| 10 interim clinical serum specimens (confirmed as positive) | 8 positive/10 (positive) |
| 10 late clinical serum specimens (confirmed as positive) | 7 positive/10 (positive) |
| 10 exceptional case specimens (confirmed as negative) | 10 negative/10 (negative) |
| 10 whole blood specimens (confirmed as negative) | 10 negative/10 (negative) |

Test strip 7: on the nitrocellulose filter membrane, the control line is coated with a goat anti-mouse IgG and/or a goat anti-chicken IgY antibody, the test line (T) is coated with a full-length S protein (antigen), and the marker pad is coated with the full-length N protein and the anti chicken IgY antibody*GOLD.

**Table 11: Test result of embodiment 6 (test strip 7)**

| Clinical specimens | Test result |
|---|---|
| 10 early clinical serum specimens (confirmed as positive) | 6 positive/10 (positive) |
| 10 interim clinical serum specimens (confirmed as positive) | 8 positive/10 (positive) |
| 10 late clinical serum specimens (confirmed as positive) | 7 positive/10 (positive) |
| 10 exceptional case specimens (confirmed as negative) | 10 negative/10 (negative) |
| 10 whole blood specimens (confirmed as negative) | 10 negative/10 (negative) |

The above test strips can conduct detection individually, or the test strip 1 can be used together with any of test strips 2-7 in pairs for detection, as shown in FIG.I to FIG.IV. From the above experiments, it can be learned that the detection of full-length S or N protein can be used as an effective supplement to only detecting S-RBD, which can effectively reduce the possibility of missing detection and comply with the actual situation.

All patents and publications mentioned in the specification of the invention indicate that these are public technologies in the field, which can be used by the invention. All patents and publications quoted herein are also listed in the references, as each publication is specifically referenced separately. The invention described herein may be implemented in the absence of any one or more elements, one or more restrictions, which are not specially specified herein. For example, the terms "including", "comprising" and "consisting of' in each embodiment can be replaced by the other two. The so-called "one" herein only means "one", while excluding or only does not mean only including one, it can also mean including more than two. The terms and expressions used here are described without limitation, and it is not intended herein to indicate that the terms and interpretations described in this document exclude any equivalent feature, but it is understood that any appropriate alteration or modification may be made to the extent of the invention and claims. It can be understood that the embodiments described in the present invention are some preferred exemplary embodiments and features. Any person skilled in the art can make some variations and changes based on the essence described in the present invention. These variations and changes are also considered within the scope of the invention and the scope limited by the independent claims and the dependent claims.

## Claims

1. A lateral flow detection device for detecting a coronavirus by immunoassay, which is **characterized in that** the detection device comprises a first test strip and a second test strip, wherein the first test strip includes an antigen to a S full-length protein of the coronavirus and/or N full-length protein of the coronavirus; the second test strip includes an antigen to a S-RBD site protein of the coronavirus; or; the first test strip includes an antibody that bind to a S full-length protein and/or N full-length protein of the coronavirus; the second test strip includes an antibody that bind to a S-RBD site protein of the coronavirus.

2. A detection device according to Claim 2, wherein an amino acid sequence of the said S full-length protein is indicated by SEQ ID NO.1, and an amino acid sequence of the said N full-length protein is indicated by SEQ ID NO.2, and an amino acid sequence of the said S-RBD site protein is indicated by SEQ ID NO.3.

3. A detection device according to Claim 2, wherein the device is **characterized in that** the first test strip or the second test strip includes a sample area, a marker area, and a detection area respectively, which are arranged in an order according to a liquid flow direction; wherein a substance coated in the marker area flows with the liquid, and the substance needs couple to a marker substance; the detection area has a test line, and the substance coated in the detection area is fixed on the test line; the said antigen may be coated in the marker area or the detection area.

4. A detection device of Claim 3, wherein the device is **characterized in that** an anti-human IgG antibody and/or an anti-human IgM antibody is immobilized on the detection area of the first test strip or the second test strip; and when the antigen is coupled to a marker substance and is treated on the marker area; or, when the antigen is immobilized in the detection area, the said anti-human IgG antibody and/or anti-human IgM antibody is coupled with the marker substance with a colored particle and is coated in the marker area.

5. A detection device of Claim 3, wherein the device is **characterized in that** a first or a second test line is arranged in the said detection area, and the first and the second test lines are respectively used for detecting any one or two of IgG and IgM in a sample.

6. A detection device of Claim 5, wherein the device is **characterized in that** a S-RBD site protein antigen on the second test strip is coated in the marker area, and the said anti-human IgG antibody and the anti-human IgM antibody are respectively immobilized on the first and second test lines of the detection area.

7. A detection device of Claim 5, wherein the device is **characterized in that** the N or/and S protein antigen on the first test strip is coated in the marker area, and the said anti-human IgG antibody and the anti-human IgM antibody are respectively immobilized on the first and second test lines of the detection area; or the anti-human IgG antibody and the anti-human IgM antibody are together immobilized on the first or the second test line of the detection area

8. A detection device of Claim 1, wherein the device is **characterized in that** the antibody comprises a first antibody or a second antibody; on the first test strip, when the first antibody to the full-length S protein and/or the first antibody to the full-length N protein is/are treated in the marker area, the second antibody to the full-length S protein and/the second antibody to N full-length protein is/are immobilized in the detection area.

9. A detection device of Claim 1, wherein the device is **characterized in that** the antibody comprises a first antibody and a second antibody, the first antibody is used to bind to an antigen in a sample, and the said first antibody is treated on the marker area; the second antibody is an antibody to the first antibody, and the second antibody is immobilized on the detection area.

10. A detection device of Claim 3, wherein the device is **characterized in that** the detection area further has a control line.

11. A detection device of Claim 3, wherein the device is **characterized in that** the said first test strip or the second test strip further comprises a water absorption area for adding a buffer solution.

12. A detection device of Claim 1, wherein the device is **characterized in that** the said coronavirus virus is a novel coronavirus.


SEQUENCE LISTING

<110>  Hangzhou Biotest Biotech Co., LTD.

<120>  LATERAL FLOW DETECTION DEVICE FOR DETECTING A CORONAVIRUS BY
       IMMUNOASSAY

<130>  MEH/116825EP1

<150>  CN 202010300484.7
<151>  2020-04-16

<150>  US 63/018,024
<151>  2020-04-30

<160>  3

<170>  PatentIn version 3.5

<210>  1
<211>  272
<212>  PRT
<213>  Coronavirus

<400>  1

Met Pro Asn Ile Thr Asn Leu Cys Pro Phe Gly Glu Val Phe Asn Ala
1               5                   10                  15

Thr Arg Phe Ala Ser Val Tyr Ala Trp Asn Arg Lys Arg Ile Ser Asn
            20                  25                  30

Cys Val Ala Asp Tyr Ser Val Leu Tyr Asn Ser Ala Ser Phe Ser Thr
        35                  40                  45

Phe Lys Cys Tyr Gly Val Ser Pro Thr Lys Leu Asn Asp Leu Cys Phe
    50                  55                  60

Thr Asn Val Tyr Ala Asp Ser Phe Val Ile Arg Gly Asp Glu Val Arg
65                  70                  75                  80

Gln Ile Ala Pro Gly Gln Thr Gly Lys Ile Ala Asp Tyr Asn Tyr Lys
                85                  90                  95

Leu Pro Asp Asp Phe Thr Gly Cys Val Ile Ala Trp Asn Ser Asn Asn
            100                 105                 110

Leu Asp Ser Lys Val Gly Gly Asn Tyr Asn Tyr Leu Tyr Arg Leu Phe
        115                 120                 125

Arg Lys Ser Asn Leu Lys Pro Phe Glu Arg Asp Ile Ser Thr Glu Ile
    130                 135                 140

Tyr Gln Ala Gly Ser Thr Pro Cys Asn Gly Val Glu Gly Phe Asn Cys
145                 150                 155                 160

Tyr Phe Pro Leu Gln Ser Tyr Gly Phe Gln Pro Thr Asn Gly Val Gly
                165                 170                 175

Tyr Gln Pro Tyr Arg Val Val Val Leu Ser Phe Glu Leu Leu His Ala
            180                 185                 190

Pro Ala Thr Val Cys Gly Pro Lys Lys Ser Thr Asn Leu Val Lys Asn
        195                 200                 205

Lys Cys Val Asn Phe Asn Phe Asn Gly Leu Thr Gly Thr Gly Val Leu
    210                 215                 220

Thr Glu Ser Asn Lys Lys Phe Leu Pro Phe Gln Gln Phe Gly Arg Asp
225                 230                 235                 240

Ile Ala Asp Thr Thr Asp Ala Val Arg Asp Pro Gln Thr Leu Glu Ile
                245                 250                 255

Leu Asp Ile Thr Pro Cys Ser Phe His His His His His His His His
            260                 265                 270

<210>  2
<211>  425
<212>  PRT
<213>  Coronavirus

<400>  2

Met His His His His His His Ser Asp Asn Gly Pro Gln Asn Gln Arg
1               5                   10                  15

Asn Ala Pro Arg Ile Thr Phe Gly Gly Pro Ser Asp Ser Thr Gly Ser
            20                  25                  30

Asn Gln Asn Gly Glu Arg Ser Gly Ala Arg Ser Lys Gln Arg Arg Pro
        35                  40                  45

Gln Gly Leu Pro Asn Asn Thr Ala Ser Trp Phe Thr Ala Leu Thr Gln
    50                  55                  60

His Gly Lys Glu Asp Leu Lys Phe Pro Arg Gly Gln Gly Val Pro Ile
65                  70                  75                  80

Asn Thr Asn Ser Ser Pro Asp Asp Gln Ile Gly Tyr Tyr Arg Arg Ala
                85                  90                  95

Thr Arg Arg Ile Arg Gly Gly Asp Gly Lys Met Lys Asp Leu Ser Pro
            100                 105                 110

Arg Trp Tyr Phe Tyr Tyr Leu Gly Thr Gly Pro Glu Ala Gly Leu Pro
        115                 120                 125

Tyr Gly Ala Asn Lys Asp Gly Ile Ile Trp Val Ala Thr Glu Gly Ala
    130                 135                 140

Leu Asn Thr Pro Lys Asp His Ile Gly Thr Arg Asn Pro Ala Asn Asn
145                 150                 155                 160

Ala Ala Ile Val Leu Gln Leu Pro Gln Gly Thr Thr Leu Pro Lys Gly
                165                 170                 175

Phe Tyr Ala Glu Gly Ser Arg Gly Gly Ser Gln Ala Ser Ser Arg Ser
            180                 185                 190

Ser Ser Arg Ser Arg Asn Ser Ser Arg Asn Ser Thr Pro Gly Ser Ser
        195                 200                 205

Arg Gly Thr Ser Pro Ala Arg Met Ala Gly Asn Gly Gly Asp Ala Ala
    210                 215                 220

Leu Ala Leu Leu Leu Leu Asp Arg Leu Asn Gln Leu Glu Ser Lys Met
225                 230                 235                 240

Ser Gly Lys Gly Gln Gln Gln Gln Gly Gln Thr Val Thr Lys Lys Ser

245                 250                 255

Ala Ala Glu Ala Ser Lys Lys Pro Arg Gln Lys Arg Thr Ala Thr Lys
        260                 265                 270

Ala Tyr Asn Val Thr Gln Ala Phe Gly Arg Arg Gly Pro Glu Gln Thr
    275                 280                 285

Gln Gly Asn Phe Gly Asp Gln Glu Leu Ile Arg Gln Gly Thr Asp Tyr
    290                 295                 300

Lys His Trp Pro Gln Ile Ala Gln Phe Ala Pro Ser Ala Ser Ala Phe
305                 310                 315                 320

Phe Gly Met Ser Arg Ile Gly Met Glu Val Thr Pro Ser Gly Thr Trp
                325                 330                 335

Leu Thr Tyr Thr Gly Ala Ile Lys Leu Asp Asp Lys Asp Pro Asn Phe
        340                 345                 350

Lys Asp Gln Val Ile Leu Leu Asn Lys His Ile Asp Ala Tyr Lys Thr
    355                 360                 365

Phe Pro Pro Thr Glu Pro Lys Lys Asp Lys Lys Lys Lys Ala Asp Glu
    370                 375                 380

Thr Gln Ala Leu Pro Gln Arg Gln Lys Lys Gln Gln Thr Val Thr Leu
385                 390                 395                 400

Leu Pro Ala Ala Asp Leu Asp Asp Phe Ser Lys Gln Leu Gln Gln Ser
                405                 410                 415

Met Ser Ser Ala Asp Ser Thr Gln Ala
        420                 425

<210>  3
<211>  1282
<212>  PRT
<213>  Coronavirus

<400>  3

Met Phe Leu Leu Thr Thr Lys Arg Thr Met Phe Val Phe Leu Val Leu
1               5                   10                  15

Leu Pro Leu Val Ser Ser Gln Cys Val Asn Leu Thr Thr Arg Thr Gln
            20                  25                  30

Leu Pro Pro Ala Tyr Thr Asn Ser Phe Thr Arg Gly Val Tyr Tyr Pro
        35                  40                  45

Asp Lys Val Phe Arg Ser Ser Val Leu His Ser Thr Gln Asp Leu Phe
    50                  55                  60

Leu Pro Phe Phe Ser Asn Val Thr Trp Phe His Ala Ile His Val Ser
65                  70                  75                  80

Gly Thr Asn Gly Thr Lys Arg Phe Asp Asn Pro Val Leu Pro Phe Asn
                85                  90                  95

Asp Gly Val Tyr Phe Ala Ser Thr Glu Lys Ser Asn Ile Ile Arg Gly
            100                 105                 110

Trp Ile Phe Gly Thr Thr Leu Asp Ser Lys Thr Gln Ser Leu Leu Ile
        115                 120                 125

Val Asn Asn Ala Thr Asn Val Val Ile Lys Val Cys Glu Phe Gln Phe
    130                 135                 140

Cys Asn Asp Pro Phe Leu Gly Val Tyr Tyr His Lys Asn Asn Lys Ser
145                 150                 155                 160

Trp Met Glu Ser Glu Phe Arg Val Tyr Ser Ser Ala Asn Asn Cys Thr
                165                 170                 175

Phe Glu Tyr Val Ser Gln Pro Phe Leu Met Asp Leu Glu Gly Lys Gln
            180                 185                 190

Gly Asn Phe Lys Asn Leu Arg Glu Phe Val Phe Lys Asn Ile Asp Gly
        195                 200                 205

Tyr Phe Lys Ile Tyr Ser Lys His Thr Pro Ile Asn Leu Val Arg Asp
    210                 215                 220

Leu Pro Gln Gly Phe Ser Ala Leu Glu Pro Leu Val Asp Leu Pro Ile
225                 230                 235                 240

Gly Ile Asn Ile Thr Arg Phe Gln Thr Leu Leu Ala Leu His Arg Ser
                245                 250                 255

Tyr Leu Thr Pro Gly Asp Ser Ser Ser Gly Trp Thr Ala Gly Ala Ala
            260                 265                 270

Ala Tyr Tyr Val Gly Tyr Leu Gln Pro Arg Thr Phe Leu Leu Lys Tyr
        275                 280                 285

Asn Glu Asn Gly Thr Ile Thr Asp Ala Val Asp Cys Ala Leu Asp Pro
    290                 295                 300

Leu Ser Glu Thr Lys Cys Thr Leu Lys Ser Phe Thr Val Glu Lys Gly
305                 310                 315                 320

Ile Tyr Gln Thr Ser Asn Phe Arg Val Gln Pro Thr Glu Ser Ile Val
                325                 330                 335

Arg Phe Pro Asn Ile Thr Asn Leu Cys Pro Phe Gly Glu Val Phe Asn
            340                 345                 350

Ala Thr Arg Phe Ala Ser Val Tyr Ala Trp Asn Arg Lys Arg Ile Ser
        355                 360                 365

Asn Cys Val Ala Asp Tyr Ser Val Leu Tyr Asn Ser Ala Ser Phe Ser
    370                 375                 380

Thr Phe Lys Cys Tyr Gly Val Ser Pro Thr Lys Leu Asn Asp Leu Cys
385                 390                 395                 400

Phe Thr Asn Val Tyr Ala Asp Ser Phe Val Ile Arg Gly Asp Glu Val
                405                 410                 415

Arg Gln Ile Ala Pro Gly Gln Thr Gly Lys Ile Ala Asp Tyr Asn Tyr
            420                 425                 430

Lys Leu Pro Asp Asp Phe Thr Gly Cys Val Ile Ala Trp Asn Ser Asn
        435                 440                 445

Asn Leu Asp Ser Lys Val Gly Gly Asn Tyr Asn Tyr Leu Tyr Arg Leu
    450                 455                 460

Phe Arg Lys Ser Asn Leu Lys Pro Phe Glu Arg Asp Ile Ser Thr Glu
465                 470                 475                 480

Ile Tyr Gln Ala Gly Ser Thr Pro Cys Asn Gly Val Glu Gly Phe Asn
                485                 490                 495

Cys Tyr Phe Pro Leu Gln Ser Tyr Gly Phe Gln Pro Thr Asn Gly Val
            500                 505                 510

Gly Tyr Gln Pro Tyr Arg Val Val Val Leu Ser Phe Glu Leu Leu His
        515                 520                 525

Ala Pro Ala Thr Val Cys Gly Pro Lys Lys Ser Thr Asn Leu Val Lys
    530                 535                 540

Asn Lys Cys Val Asn Phe Asn Phe Asn Gly Leu Thr Gly Thr Gly Val
545                 550                 555                 560

Leu Thr Glu Ser Asn Lys Lys Phe Leu Pro Phe Gln Gln Phe Gly Arg
                565                 570                 575

Asp Ile Ala Asp Thr Thr Asp Ala Val Arg Asp Pro Gln Thr Leu Glu
            580                 585                 590

Ile Leu Asp Ile Thr Pro Cys Ser Phe Gly Gly Val Ser Val Ile Thr
        595                 600                 605

Pro Gly Thr Asn Thr Ser Asn Gln Val Ala Val Leu Tyr Gln Asp Val
    610                 615                 620

Asn Cys Thr Glu Val Pro Val Ala Ile His Ala Asp Gln Leu Thr Pro
625                 630                 635                 640

Thr Trp Arg Val Tyr Ser Thr Gly Ser Asn Val Phe Gln Thr Arg Ala
                645                 650                 655

Gly Cys Leu Ile Gly Ala Glu His Val Asn Asn Ser Tyr Glu Cys Asp
            660                 665                 670

Ile Pro Ile Gly Ala Gly Ile Cys Ala Ser Tyr Gln Thr Gln Thr Asn
        675                 680                 685

Ser Pro Arg Arg Ala Arg Ser Val Ala Ser Gln Ser Ile Ile Ala Tyr
    690                 695                 700

Thr Met Ser Leu Gly Ala Glu Asn Ser Val Ala Tyr Ser Asn Asn Ser
705                 710                 715                 720

Ile Ala Ile Pro Thr Asn Phe Thr Ile Ser Val Thr Thr Glu Ile Leu
                725                 730                 735

Pro Val Ser Met Thr Lys Thr Ser Val Asp Cys Thr Met Tyr Ile Cys
            740                 745                 750

Gly Asp Ser Thr Glu Cys Ser Asn Leu Leu Leu Gln Tyr Gly Ser Phe
        755                 760                 765

Cys Thr Gln Leu Asn Arg Ala Leu Thr Gly Ile Ala Val Glu Gln Asp
    770                 775                 780

Lys Asn Thr Gln Glu Val Phe Ala Gln Val Lys Gln Ile Tyr Lys Thr
785                 790                 795                 800

Pro Pro Ile Lys Asp Phe Gly Gly Phe Asn Phe Ser Gln Ile Leu Pro
                805                 810                 815

Asp Pro Ser Lys Pro Ser Lys Arg Ser Phe Ile Glu Asp Leu Leu Phe
            820                 825                 830

Asn Lys Val Thr Leu Ala Asp Ala Gly Phe Ile Lys Gln Tyr Gly Asp
        835                 840                 845

Cys Leu Gly Asp Ile Ala Ala Arg Asp Leu Ile Cys Ala Gln Lys Phe
    850                 855                 860

Asn Gly Leu Thr Val Leu Pro Pro Leu Leu Thr Asp Glu Met Ile Ala
865                 870                 875                 880

Gln Tyr Thr Ser Ala Leu Leu Ala Gly Thr Ile Thr Ser Gly Trp Thr
                885                 890                 895

Phe Gly Ala Gly Ala Ala Leu Gln Ile Pro Phe Ala Met Gln Met Ala
            900                 905                 910

Tyr Arg Phe Asn Gly Ile Gly Val Thr Gln Asn Val Leu Tyr Glu Asn
        915                 920                 925

Gln Lys Leu Ile Ala Asn Gln Phe Asn Ser Ala Ile Gly Lys Ile Gln
    930                 935                 940

Asp Ser Leu Ser Ser Thr Ala Ser Ala Leu Gly Lys Leu Gln Asp Val
945                 950                 955                 960

Val Asn Gln Asn Ala Gln Ala Leu Asn Thr Leu Val Lys Gln Leu Ser
                965                 970                 975

Ser Asn Phe Gly Ala Ile Ser Ser Val Leu Asn Asp Ile Leu Ser Arg
            980                 985                 990

Leu Asp Lys Val Glu Ala Glu Val  Gln Ile Asp Arg Leu  Ile Thr Gly
        995                 1000                1005

Arg Leu  Gln Ser Leu Gln Thr  Tyr Val Thr Gln Gln  Leu Ile Arg
    1010                1015                1020

Ala Ala  Glu Ile Arg Ala Ser  Ala Asn Leu Ala Ala  Thr Lys Met
    1025                1030                1035

Ser Glu  Cys Val Leu Gly Gln  Ser Lys Arg Val Asp  Phe Cys Gly
    1040                1045                1050

Lys Gly  Tyr His Leu Met Ser  Phe Pro Gln Ser Ala  Pro His Gly
    1055                1060                1065

Val Val  Phe Leu His Val Thr  Tyr Val Pro Ala Gln  Glu Lys Asn
    1070                1075                1080

Phe Thr Thr Ala Pro Ala Ile Cys His Asp Gly Lys Ala His Phe
    1085                1090                1095

Pro Arg Glu Gly Val Phe Val Ser Asn Gly Thr His Trp Phe Val
    1100                1105                1110

Thr Gln Arg Asn Phe Tyr Glu Pro Gln Ile Ile Thr Thr Asp Asn
    1115                1120                1125

Thr Phe Val Ser Gly Asn Cys Asp Val Val Ile Gly Ile Val Asn
    1130                1135                1140

Asn Thr Val Tyr Asp Pro Leu Gln Pro Glu Leu Asp Ser Phe Lys
    1145                1150                1155

Glu Glu Leu Asp Lys Tyr Phe Lys Asn His Thr Ser Pro Asp Val
    1160                1165                1170

Asp Leu Gly Asp Ile Ser Gly Ile Asn Ala Ser Val Val Asn Ile
    1175                1180                1185

Gln Lys Glu Ile Asp Arg Leu Asn Glu Val Ala Lys Asn Leu Asn
    1190                1195                1200

Glu Ser Leu Ile Asp Leu Gln Glu Leu Gly Lys Tyr Glu Gln Tyr
    1205                1210                1215

Ile Lys Trp Pro Trp Tyr Ile Trp Leu Gly Phe Ile Ala Gly Leu
    1220                1225                1230

Ile Ala Ile Val Met Val Thr Ile Met Leu Cys Cys Met Thr Ser
    1235                1240                1245

Cys Cys Ser Cys Leu Lys Gly Cys Cys Ser Cys Gly Ser Cys Cys
    1250                1255                1260

Lys Phe Asp Glu Asp Asp Ser Glu Pro Val Leu Lys Gly Val Lys
    1265                1270                1275

Leu His  Tyr Thr
    1280